# EUROPEAN PATENT APPLICATION

(11) **EP 2 460 796 A1**
(43) Date of publication of application: **06.06.2012**
(21) Application number: 10804304.3
(22) Date of filing: 21.07.2010
(51) Int. Cl.: C07D 209/94, G03G 5/06

(54) **INDOLE DERIVATIVE**

(30) Priority: 28.07.2009 JP 2009174976
(71) Applicant: Hodogaya Chemical Co., Ltd., Tokyo 105-0011 (JP)
(72) Inventor: NUMAZAWA, Shigetaka, Tsukuba-shi Ibaraki 305-0841 (JP); ABE, Katsumi, Koriyama-shi Fukushima 963-8802 (JP); IHARA, Kiyotaka, Koriyama-shi Fukushima 963-8802 (JP); NAKAJIMA, Takehiro, Koriyama-shi Fukushima 963-8802 (JP); KOIKE, Makoto, Koriyama-shi Fukushima 963-8802 (JP)
(74) Representative: Ehlich, Hendrik
(86) International application number: PCT/JP2010/062262
(87) International publication number: WO 2011/013558

(57) **Abstract**

[Problems] To provide a novel compound having a high carrier mobility and is useful as a charge transporting agent which not only makes it possible to stably form a photosensitive layer without precipitating crystals or without developing pinholes when the photosensitive layer is being formed but also makes it possible to form an organic photosensitive material for electrophotography of a high sensitivity and a low residual potential.

[Means for Solution] An indole derivative represented by the following general formula (1), 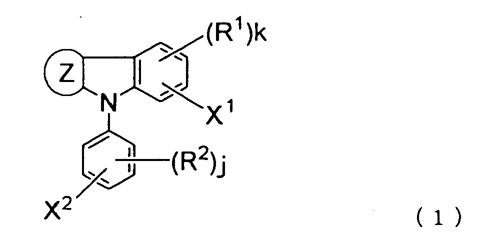 wherein R¹ and R² are alkyl groups, k is an integer of 0 to 3, j is an integer of 0 to 4, a ring Z is a 5- to 6-membered ring and is, specifically, a cyclopentane ring, and X¹ and X² are hydrocarbon groups having at least one ethylenically unsaturated bond.

## Description

### Technical Field:

This invention relates to indole derivatives having a high carrier mobility and being useful as a charge transporting agent for a photosensitive material for electrophotography.

### Background Art:

As inorganic photoconductive materials, there have been known amorphous silicon, amorphous selenium, cadmium sulfide, zinc oxide and the like. Inorganic photosensitive materials formed by using such inorganic photoconductive materials have been widely used in the field of electrophotography. However, selenium and cadmium sulfide must be recovered as toxic substances, selenium has poor resistance against the heat since it is crystallized by heating, cadmium sulfide and zinc oxide have poor resistance against the moisture, and zinc oxide has poor resistance against the printing. As the photoconductive material, therefore, an organic photosensitive material is now becoming a mainstream comprising an electrically conducting substrate on which is provided an organic photosensitive layer containing a charge generating agent and a charge transporting agent.
As organic photosensitive material, there have been known the one of the single layer type in which a photosensitive layer formed on the electrically conducting substrate contains a charge generating agent and a charge transporting agent that are dispersed in a resin binder, and the one of the lamination type in which the photosensitive layer comprises a charge generating layer containing the charge generating agent dispersed in a resin binder and a charge transporting layer containing the charge transporting agent dispersed in a resin binder. The organic photosensitive material of either type has such advantages that it is lighter in weight than the inorganic photosensitive materials and enables the photosensitive layer to be easily formed and, further, offers such an advantage that it little affects the environment.

In the above organic photosensitive material for electrophotography, the charge transporting agent must satisfy such properties as efficiently receiving carriers (positive charge or negative charge) generated by the charge generating agent upon the irradiation with light when an electric field is applied, quickly migrating the carriers in the photosensitive layer and quickly extinguishing the electric charge on the surface of the photosensitive layer. The rate of migration of the carriers per a unit electric field is called carrier mobility, and a high carrier mobility means that the carriers migrate quickly in the photosensitive layer (or in the charge transporting layer). The carrier mobility is specific to a compound used as the charge transporting agent. As the charge transporting agent, therefore, it is necessary to use a compound having a high carrier mobility.

Further, the charge transporting agent and the charge generating agent form the photosensitive layer by being dissolved together with a resin binder in an organic solvent and being applied and dried (removal of the organic solvent). Therefore, the charge transporting agent must satisfy such properties as forming a homogeneous photosensitive layer without precipitating crystals and without developing pinholes. If crystals are locally precipitated or pinholes are formed in the photosensitive layer, dielectric breakdown occurs in such portions, and the image defect occurs when the image is formed by the electrophotographic method.

As described above, the charge transporting agent must satisfy a variety of properties. Many kinds of compounds have heretofore been proposed as charge transporting agents (see patent documents 1 to 14). In particular, patent documents 15 and 16 propose indole derivatives expressed by specific general formulas as charge transporting agents. Of them, the patent document 16 discloses an indole derivative expressed by the following formula that is used as a charge transporting agent.

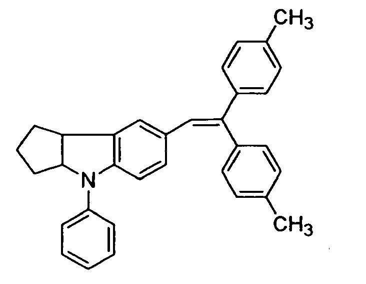

### Prior Art Documents:

### Patent documents:

Patent document 1: JP-B-58-32372
Patent document 2: JP-A-1-142642
Patent document 3: JP-A-5-088389
Patent document 9: JP-B-7-021646
Patent document 5: JP-B-5-019701
Patent document 6: JP-B-55-042380
Patent document 7: JP-A-57-101844
Patent document 8: JP-A-54-150128
Patent document 9: JP-A-61-023154
Patent document 10: JP-B-55-042380
Patent document 11: JP-A-60-340999
Patent document 12: JP-A-61-023154
Patent document 13: JP-B-58-032372
Patent document 14: U.S. Patent No. 3873312
Patent document 15: JP-A-3-075660
Patent document 16: JP-A-2000-098640

### Outline of the Invention:

### Problems that the Invention is to Solve:

Many compounds proposed by the above patent documents as charge transporting agents have charge mobilities which are high to some extent. When the organic photosensitive materials are prepared by using these compounds as charge transporting agents, however, homogeneous photosensitive layers cannot be easily formed due to the precipitation of crystals or formation of pinholes. Even if the photosensitive layers were formed, the surface potential of the photosensitive layer formed by the main electric charge cannot be fully maintained, the surface potential cannot be fully extinguished after the irradiation with light (after exposed to image), leaving such problems as low sensitivity and high residual potential, and further improvements are required.

It is, therefore, an object of the present invention to provide a novel compound being useful as a charge transporting agent, which has a high carrier mobility, and which not only makes it possible to stably form a photosensitive layer without precipitating crystals or without developing pinholes when the photosensitive layer is being formed, but also makes it possible to form an organic photosensitive material for electrophotography of a high sensitivity and a low residual potential.
Another object of the present invention is to provide a charge transporting agent comprising the above compound and an organic photosensitive material for electrophotography containing the above charge transporting agent in the photosensitive layer.

### Means for Solving the Problems:

According to the present invention, there is provided an indole derivative represented by the following general formula (1), 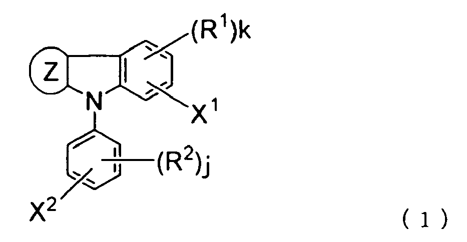 wherein,
R¹ and R² may be same or different, and are groups selected from the group consisting of an alkyl group having 1 to 6 carbon atoms; an alkoxy group having 1 to 6 carbon atoms; a halogen atom; an aromatic hydrocarbon group; an aromatic heterocyclic group; a condensed polycyclic aromatic group; and a di-substituted amino group which has, as a substituent, an alkyl group with 1 to 6 carbon atoms, an alkenyl group with 1 to 6 carbon atoms, an aralkyl group, an aromatic hydrocarbon group or an aromatic heterocyclic group;,
k is an integer of 0 to 3,
j is an integer of 0 to 4,
(when k or j is an integer of not smaller than 2, a plurality of R¹s or R²s may be different from each other),
a ring Z bonded to the indoline ring is a 5- to 8-membered ring having no unsaturated bond in the ring, and may have nitrogen and/or oxygen as ring-constituting atoms,
X¹ is a monovalent group represented by the following general formula (1a),

   -(-CR³=CR⁴-)ₘ-CR⁵=CR⁶R⁷ (1a)

   wherein,
   m is 0 or 1, and
   R³ to R³ may be same or different, and are hydrogen atoms, alkyl groups having 1 to 6 carbon atoms, alkoxy groups having 1 to 6 carbon atoms, aromatic hydrocarbon groups, aromatic heterocyclic groups or condensed polycyclic aromatic groups, R⁶ and R⁷ together may form a ring, and when R⁶ is a hydrogen atom or an alkyl group, R⁷ is an aromatic hydrocarbon group, an aromatic heterocyclic group or a condensed polycyclic aromatic group, and
   X² is a monovalent group represented by the following general formula (1b),

      -(-CR⁸=CR⁹-)ₙ-CR¹⁰=CR¹¹R¹² (1b)

      wherein,
      n is 0 or 1, and
      R⁸ to R¹² may be same or different, and are hydrogen atoms, alkyl groups having 1 to 6 carbon atoms, alkoxy groups having 1 to 6 carbon atoms, aromatic hydrocarbon groups, aromatic heterocyclic groups or condensed polycyclic aromatic groups, R¹¹ and R¹² together may form a ring, and when R¹¹ is a hydrogen atom or an alkyl group, R¹² is an aromatic hydrocarbon group, an aromatic heterocyclic group or a condensed polycyclic aromatic group.

In the indole derivative of the invention, it is desired that:
(A) In the general formula (1), the ring Z is a 5-membered ring with carbon atoms as ring-constituting atoms;
(B) In the general formula (1a) representing the group X¹, m is 0 and in the general formula (1b) representing the group X², n is 0;
(C) In the general formula (1a) representing the group X¹, m is 0 and in the general formula (1b) representing the group X², n is 1; and
(D) In the general formula (1), k and j are 0s.

According to the present invention, there is, further, provided a charge transporting agent comprising the indole derivative.
According to the present invention, further, there is provided an organic photosensitive material for electrophotography comprising an organic photosensitive layer provided on an electrically conducting substrate, the organic photosensitive layer containing the indole derivative as a charge transporting agent.

In the organic photosensitive material for electrophotography, it is desired that:
(E) The organic photosensitive layer is a lamination type photosensitive layer which comprises a charge generating layer in which the charge generating agent is dispersed in a resin binder and a charge transporting layer in which the charge transporting agent is dispersed in a resin binder; or
(F) The organic photosensitive layer is a single photosensitive layer in which the charge generating agent and the charge transporting agent are dispersed in a resin binder.

### Effects of the Invention:

The indole derivative of the invention represented by the above general formula (1) is a novel compound having a high carrier mobility. The indole derivative is very useful as a charge transporting agent for the production of an organic photosensitive material for electrophotography.
Further, the organic photosensitive material containing the indole derivative as the charge transporting agent in the photosensitive layer causes little precipitation of crystals or little occurrence of pinholes at the time of forming the photosensitive layer, features a high sensitivity and a low residual potential, further, permits a little fluctuation in the surface potential, a little decrease in the sensitivity and a little accumulation of residual potential when images are formed repeatedly by the electrophotography method, and provides excellent durability.

### Brief Description of the Drawings:

[Fig. 1] shows an IR spectrum of a compound (exemplified compound 4) of Example 1 of the invention.
[Fig. 2] shows an IR spectrum of a compound (exemplified compound 5) of Example 2 of the invention.
[Fig. 3] shows an IR spectrum of a compound (exemplified compound 22) of Example 3 of the invention.

### Description of Preferred Embodiments

### <Indole derivatives>

An indole derivative of the invention is expressed by the following general formula (1),

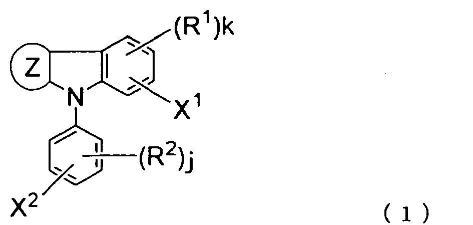

In the general formula (1), k is an integer of 0 to 3 representing the number of the groups R¹, and j is an integer of 0 to 4 representing the number of the groups R². Further, R¹, R², ring Z, X¹ and X² are as described below.

### (Groups R¹ and R²)

The groups R¹ and R² may be the same or different ones, and are each any one of alkyl group, alkoxy group, halogen atom, aromatic hydrocarbon group, aromatic heterocyclic group, condensed polycyclic aromatic group or di-substituted amino group.

The alkyl group has carbon atoms in a number over a range of 1 to 6, and may be either in the form of a straight chain or a branched form.
Concrete examples of the alkyl group include methyl group, ethyl group, propyl group, butyl group, hexyl group, tert-butyl group and isopropyl group.

The alkoxy group has carbon atoms in a number over a range of 1 to 6, and may be either in the form a straight-chain or a branched form.
Concrete examples of the alkoxy group include methoxy group, ethoxy group and propyloxy group.

As the halogen atom, there can be exemplified fluorine atom, chlorine atom, bromine atom and iodine atom.

As the aromatic hydrocarbon group or the condensed polycyclic aromatic group, there can be exemplified phenyl group, naphthenyl group, anthracenyl group and pyrenyl group.

As the aromatic heterocyclic group, there can be exemplified pyridyl group, pyrolyl group, thienyl group, furyl group, carbazolyl group and pyronyl group.

The di-substituted amino group is the one in which two substituents are bonded to the nitrogen atom thereof. As such substituents, there can be exemplified the alkyl group (either a straight chain one or a branched one) having 1 to 6 carbon atoms exemplified above, aromatic hydrocarbon group or aromatic heterocyclic group. In addition to the above, there can be exemplified an alkenyl group (which may be a straight chain one or a branched one, such as allyl group) having 1 to 6 carbon atoms, and aralkyl group (e.g., benzyl group or phenetyl group).
As concrete examples of the di-substituted amino group having such substituents, there can be exemplified dimethylamino group, diethylamino group, diphenylamino group, dinaphthylamino group, dibenzylamino group, diphenetylamino group, dipiridylamino group, dithienylamino group and diallylamino group.

When the groups R¹ or the groups R² are present in a plurality of numbers (k or j is an integer of not smaller than 2), the plurality of the groups R¹ or the groups R² may be different from each other.

The above alkyl group, alkoxy group, aromatic hydrocarbon group, aromatic heterocyclic group, condensed polycyclic aromatic group, and substituents possessed by the di-substituted amino group may, further, have another substituent.
As such a substituent, there can be exemplified the following substituents so far as they satisfy a predetermined number of carbon atoms.
Hydroxyl group;
Halogen atom such as fluorine atom, chlorine atom, bromine atom or iodine atom;
Alkyl group (either straight chain or branched) having 1 to 6 carbon atoms, such as methyl group, ethyl group, propyl group, butyl group, hexyl group or isopropyl group;
Straight chain or branched alkoxy group having 1 to 6 carbon atoms, such as methoxy group, ethoxy group or propyloxy group;
Alkenyl group such as allyl group;
Aralkyl group such as benzyl group, naphthylmethyl group or phenetyl group;
Aryloxy group such as phenoxy group or tolyloxy group;
Arylalkoxyl group such as benzyloxy group or phenetyloxy group;
Aromatic hydrocarbon group or condensed polycyclic aromatic group, such as phenyl group, naphthyl group, anthracenyl group or pylenyl group;
aromatic heterocyclic group such as pyridyl group, pyrrolyl group, thienyl group, furyl group, carbazolyl group or pyrronyl group;
Arylvinyl group such as styryl group or naphthylvinyl group;
Acyl group such as acetyl group or benzoyl group;
Dialkylamino group such as dimethylamino group or diethylamino group;
Di-substituted amino group substituted with aromatic hydrocarbon group or condensed polycyclic aromatic group, such as diphenylamino group or dinaphthylamino group;
Diaralkylamino group such as dibenzylamino group or diphenetylamino group;
Di-substituted amino group substituted with an aromatic heterocyclic group, such as dipyridylamino group or dithienylamino group; and
Dialkenylamino group such as diallylamino group.
When the above substituents are present in a plurality of numbers, these substituents may be condensed with each other and may form a carbocyclic group or a heterocyclic ring group that contains oxygen atom, sulfur atom or nitrogen atom via a single bond or via a methylene group, ethylene group, carbonyl group, vinylidene group or ethylenylene group. These substituents may, further, have another substituent.

In the above group R¹ and group R², the specifically desired group is a methyl group or a phenyl group.

### (Group X¹)

In the general formula (1), the group X¹ is a monovalent group represented by the following formula (1a).

-(-CR³=CR⁴-)ₘ-CR⁵=CR⁶R⁷ (1a)

In the general formula (1a), m is the number of a recurring unit (-CR³=CR⁴-) and is 0 or 1.

The groups R³ to R⁷ may be same or different, and are hydrogen atoms, straight chain or branched alkyl groups having 1 to 6 carbon atoms, straight chain or branched alkoxy groups having 1 to 6 carbon atoms, aromatic hydrocarbon groups, aromatic heterocyclic groups or condensed polycyclic aromatic groups. Concrete examples of the groups R³ to R⁷ may be the same groups as those exemplified for the above groups R¹ and R². These groups R³ to R⁷, too, may have the same substituents as those possessed by the above groups R¹ and R².

Of the above groups R³ to R⁷, when R⁶ is a hydrogen atom or an alkyl group, R⁷ is an aromatic hydrocarbon group, aromatic heterocyclic group or condensed polycyclic aromatic group.

Further, R⁶ and R⁷ together may form a ring. For instance, R⁶ and R⁷ may be bonded together directly, or bonded together via methylene group, ethylene group, carbonyl group, vinylidene group or ethylenylene group to form a carbocyclic group or a heterocyclic group that contains oxygen atom, sulfur atom or nitrogen atom.

### (Group X²)

In the general formula (1), the group X² is a monovalent group represented by the following formula (1b).

-(-CR⁸=CR⁹-)ₙ-CR¹⁰=CR¹¹R¹² (1b)

In the general formula (1b), n is the number of a recurring unit (-CR⁸=CR⁹-) and is 0 or 1.

The groups R⁸ to R¹² may be same or different, and are hydrogen atoms, straight chain or branched alkyl groups having 1 to 6 carbon atoms, straight chain or branched alkoxy groups having 1 to 6 carbon atoms, aromatic hydrocarbon groups, aromatic heterocyclic groups or condensed polycyclic aromatic groups. Concrete examples of the groups R⁸ to R¹² may be the same groups as those exemplified for the above groups R¹ and R².
That is, as the alkyl group having 1 to 6 carbon atoms, there can be exemplified methyl group, ethyl group, propyl group, butyl group, hexyl group, tert-butyl group and isopropyl group.
As the alkoxy group having 1 to 6 carbon atoms, there can be exemplified methoxy group, ethoxy group and propyloxy group.
As the aromatic hydrocarbon groups or condensed polycyclic aromatic groups, there can be exemplified phenyl group, naphthyl group, anthracenyl group and pyrenyl group.
As the aromatic heterocyclic group, there can be exemplified pyridyl group, pyrrolyl group, thienyl group, furyl group, carbazolyl group and pyronyl group.
These groups R⁸ to R¹², too, may have the same substitutes as those possessed by the above groups R¹ and R².

Of the above groups R⁸ to R¹², when R¹¹ is a hydrogen atom or an alkyl group, R¹² is an aromatic hydrocarbon group, aromatic heterocyclic group or condensed polycyclic aromatic group.

Further, of these groups, R¹¹ and R¹² together may form a ring like the above groups R⁶ and R⁷. For instance, R¹¹ and R¹² may be bonded together directly or via methylene group, ethylene group, carbonyl group, vinylidene group or ethylenylene group to form a carbocyclic group or a heterocyclic group that contains oxygen atom, sulfur atom or nitrogen atom.

In the invention, the group X² represented by the above general formula (1b) is a group that is particularly important for producing an organic photosensitive material which maintains a high carrier mobility and features a high sensitivity and a low residual potential. For example, the indole derivatives proposed by the above patent documents 15 and 16 do not have the above group X², and their carrier mobilities are lower than that of the indole derivative of the present invention and, besides, their photosensitive materials that are obtained exhibit low sensitivities (large half-decay exposures) and high residual potentials (see Examples and Comparative Examples appearing later).

### (Ring Z)

In the general formula (1), the ring Z bonded to the indoline ring is a 5- to 8-membered ring having no unsaturated bond in the ring, and is a carbon ring such as cyclopentane ring, cyclohexane ring, cycloheptane ring or cyclooctane ring, or a heterocyclic ring in which carbon atoms in the carbon ring are partly substituted with oxygen atoms, sulfur atoms and/or nitrogen atoms. Specifically, a carbon ring of a 5- to 6-membered ring is preferred and the cyclopentane ring is most preferred.
Further, the ring Z may have a substituent. The substituent may be the same as those substituents exemplified for the groups R³ to R¹² in the above general formulas (1a) and (1b).

Described below are concrete examples of the indole derivative represented by the above general formula (1).
In the following formulas, the value of m in the general formula (1a) and the value of n in the general formula (1b) are also described.

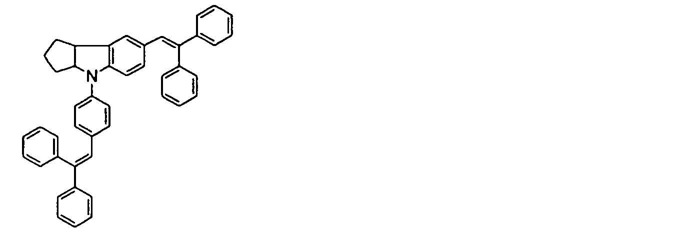

(Example compound 1, m=0, n=0)

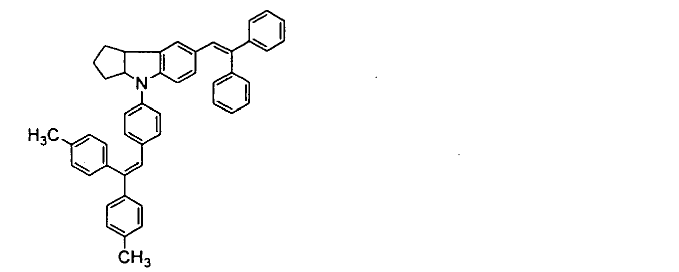

(Example compound 2, m=0, n=0)

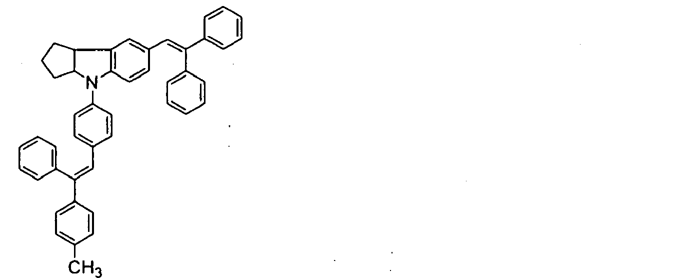

(Example compound 3, m=0, n=0)

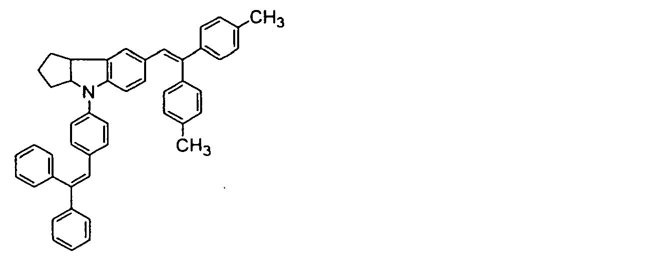

(Example compound 4, m=0, n=0)

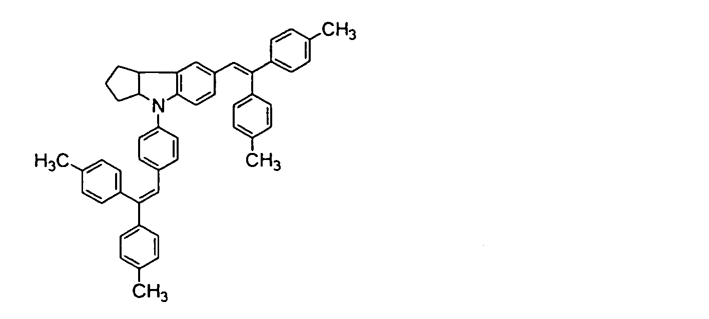

(Example compound 5, m=0, n=0)

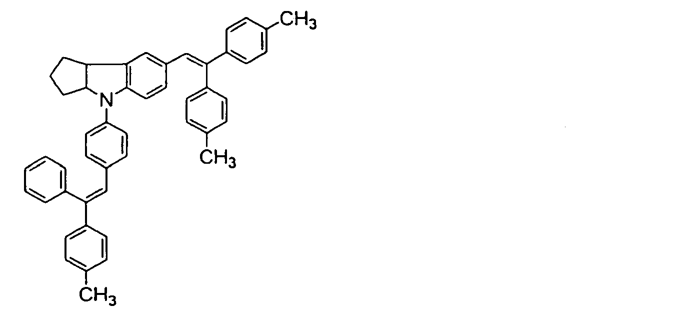

(Example compound 6, m=0, n=0)

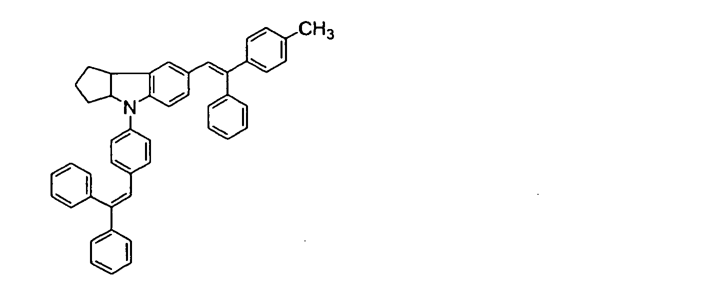

(Example compound 7, m=0, n=0)

1 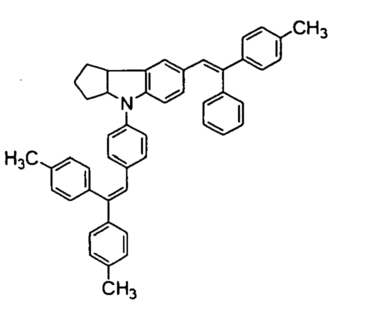 (Example compound 8, m=0, n=0)

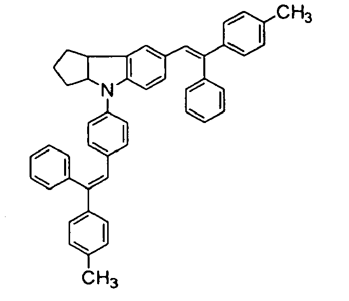

(Example compound 9, m=0, n=0)

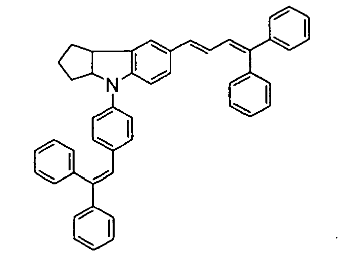

(Example compound 10, m=1, n=0)

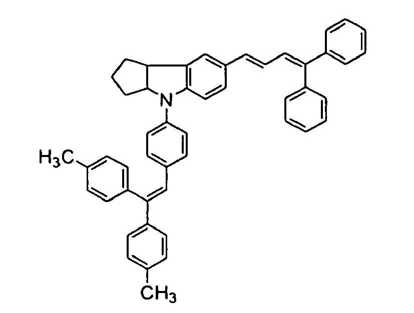

(Example compound 11, m=1, n=0)

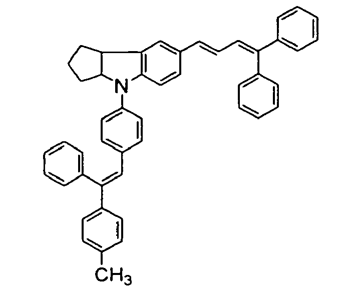

(Example compound 12, m=1, n=0)

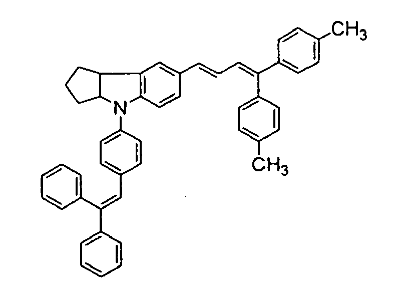

(Example compound 13, m=1, n=0)

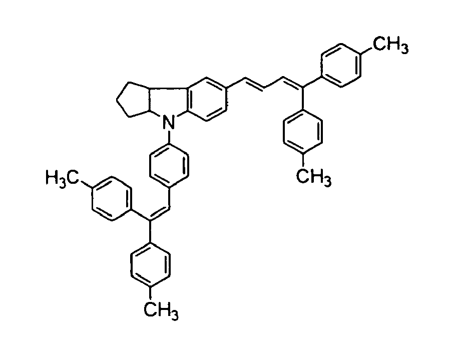

(Example compound 14, m=1, n=0)

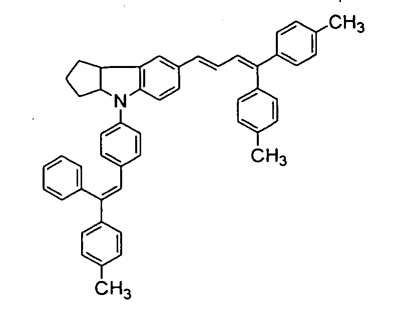

(Example compound 15, m=1, n=0)

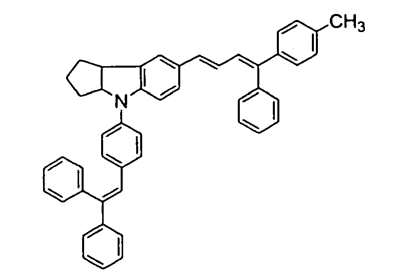

(Example compound 16, m=1, n=0)

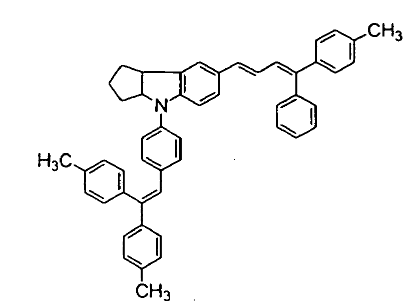

(Example compound 17, m=1, n=0)

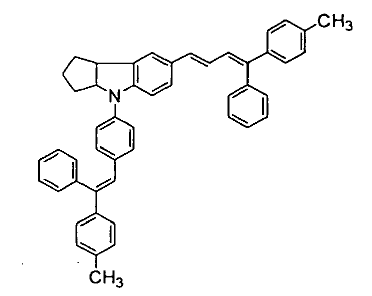

(Example compound 18, m=1, n=0)

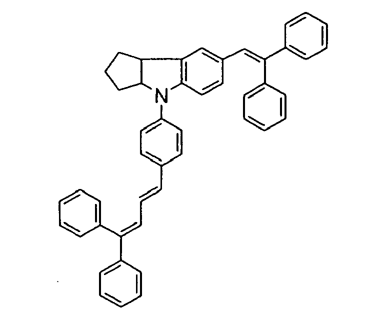

(Example compound 19, m=0, n=1)

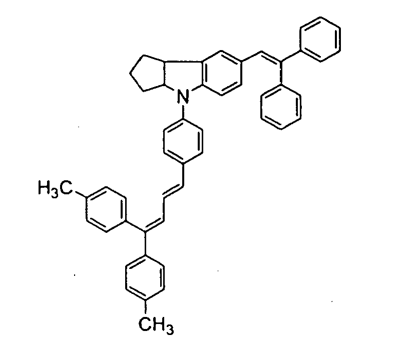

(Example compound 20, m=0, n=1)

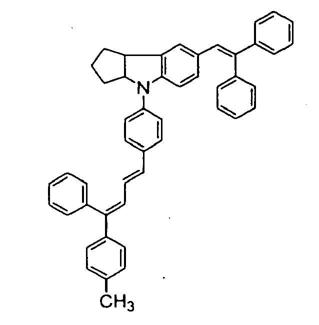

(Example compound 21, m=0, n=1)

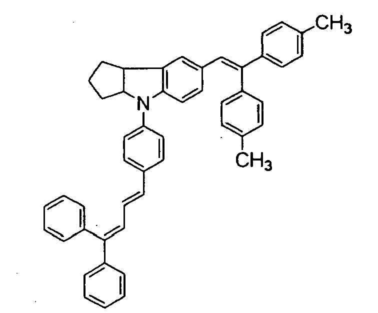

(Example compound 22, m=0, n=1)

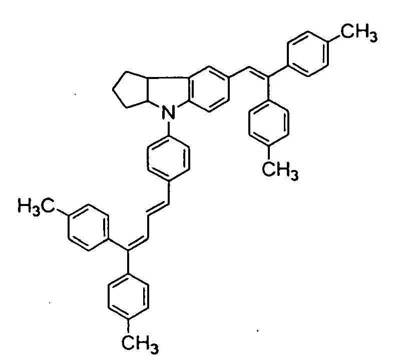

(Example compound 23, m=0, n=1)

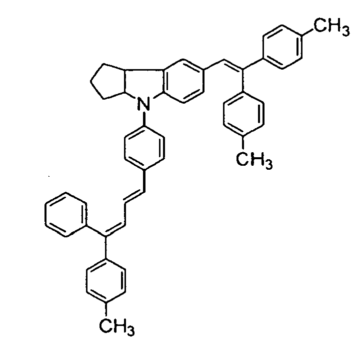

(Example compound 24, m=0, n=1)

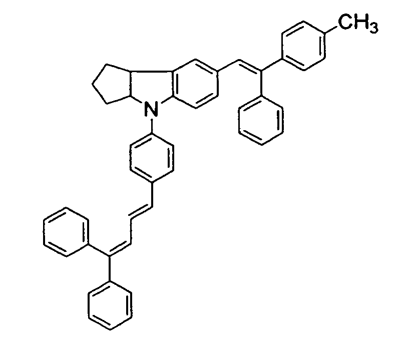

(Example compound 25, m=0, n=1)

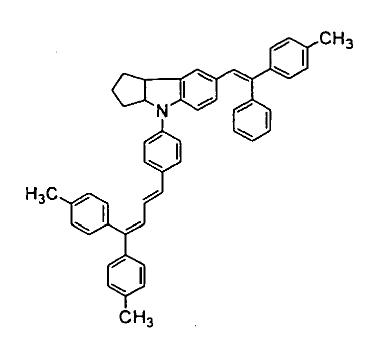

(Example compound 26, m=0, n=1)

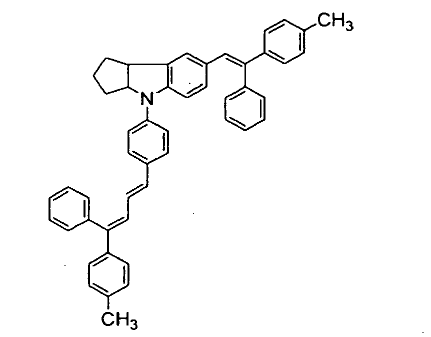

(Example compound 27, m=0, n=1)

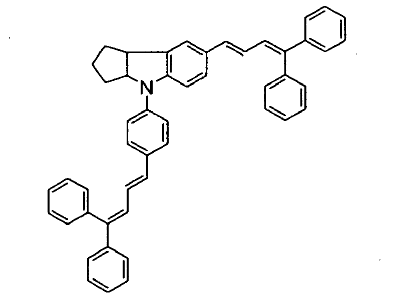

(Example compound 28, m=1, n=1)

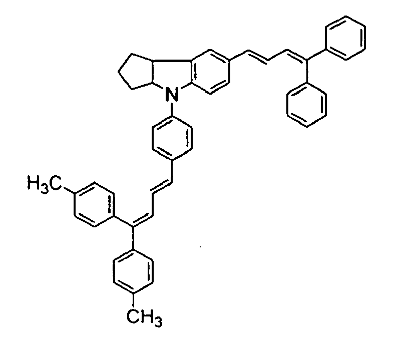

(Example compound 29, m=1, n=1)

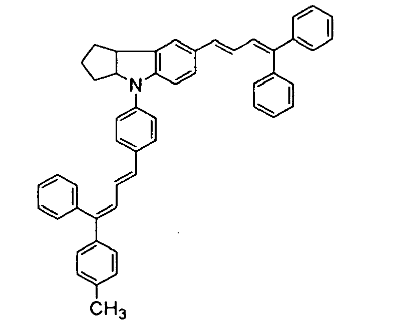

(Example compound 30, m=1, n=1)

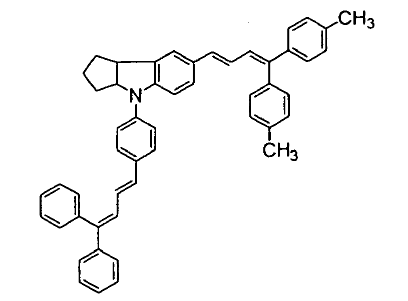

(Example compound 31, m=1, n=1)

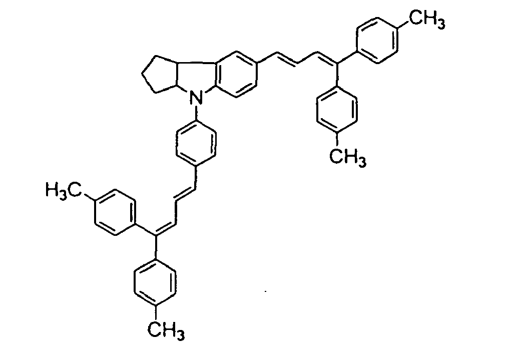

(Example compound 32, m=1, n=1)

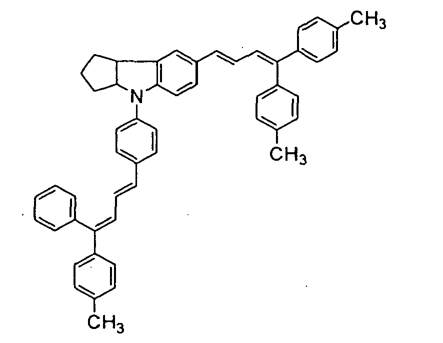

(Example compound 33, m=1, n=1)

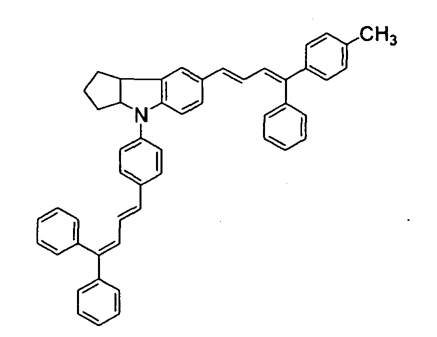

(Example compound 34, m=1, n=1)

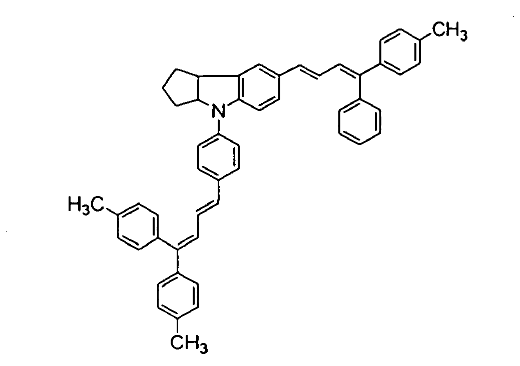

(Example compound 35, m=1, n=1)

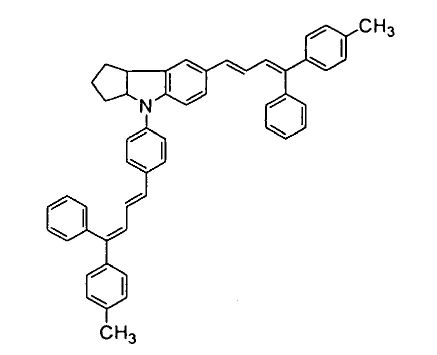

(Example compound 36, m=1, n=1)

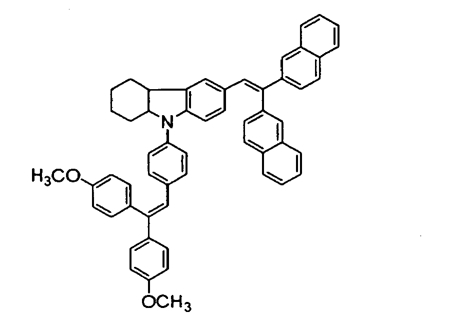

(Example compound 37, m=0, n=0)

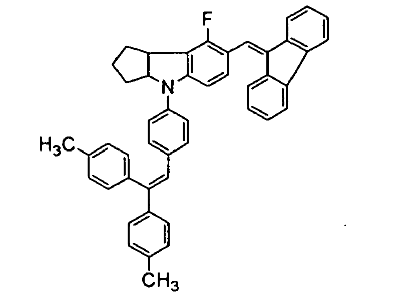

(Example compound 38, m=0, n=0)

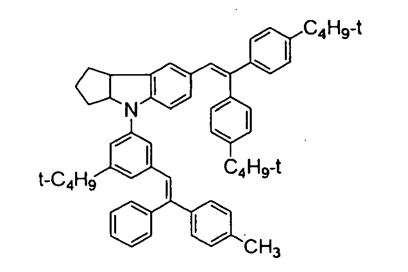

(Example compound 39, m=0, n=0)

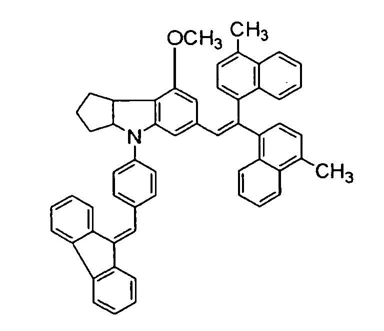

(Example compound 40, m=0, n=0)

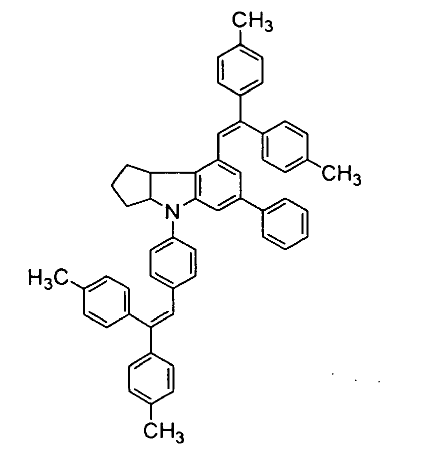

(Example compound 41, m=0, n=0)

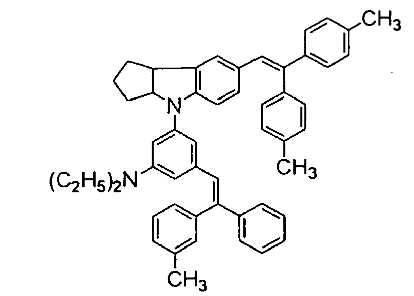

(Example compound 42, m=0, n=0)

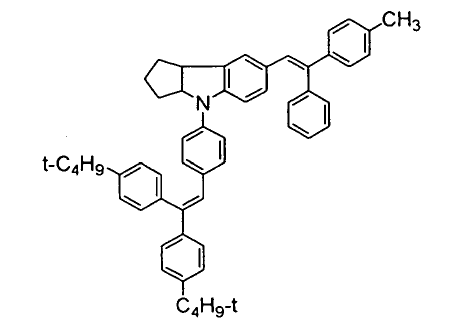

(Example compound 43, m=0, n=0)

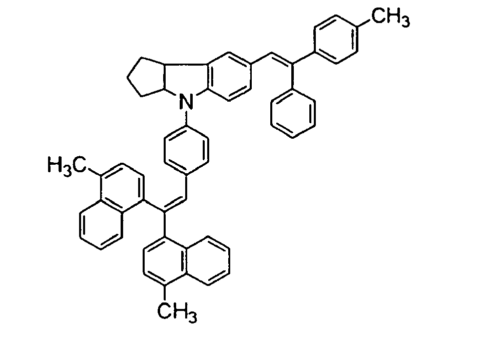

(Example compound 44, m=0, n=0)

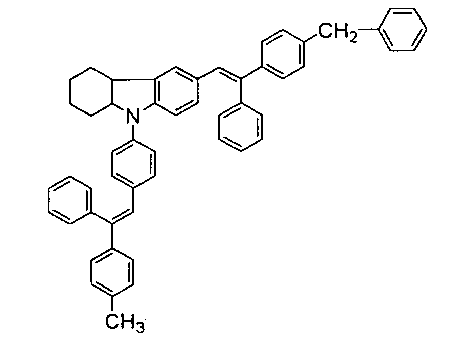

(Example compound 45, m=0, n=0)

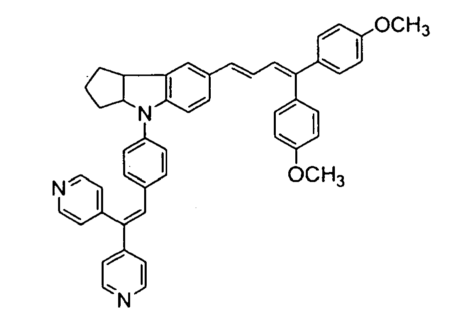

(Example compound 46, m=1, n=0)

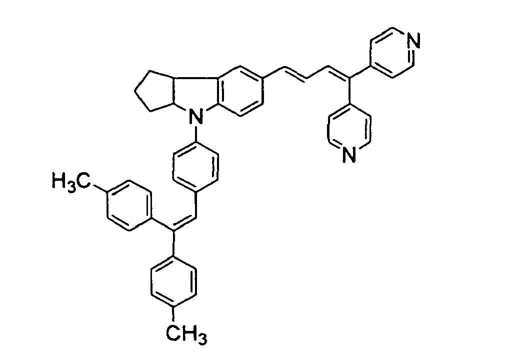

(Example compound 47, m=1, n=0)

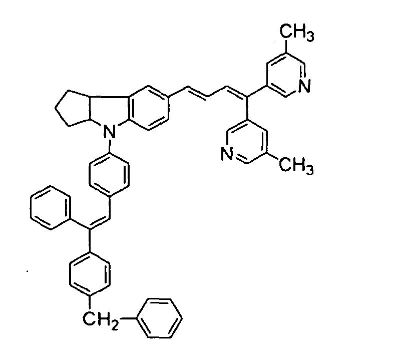

(Example compound 48, m=1, n=0)

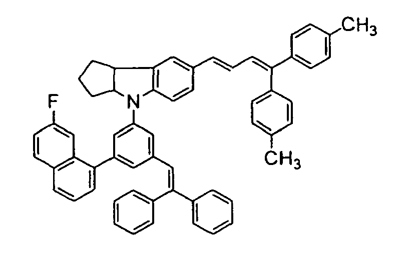

(Example compound 49, m=1, n=0)

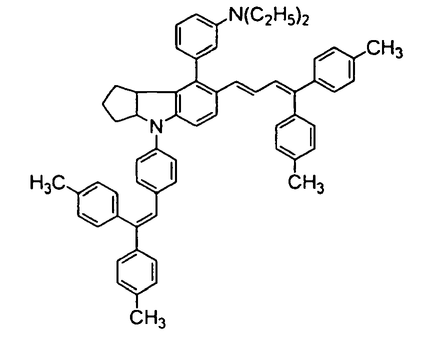

(Example compound 50, m=1, n=0)

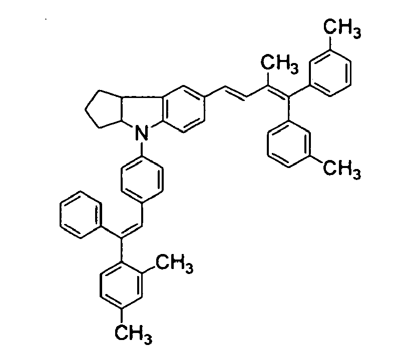

(Example compound 51, m=1, n=0)

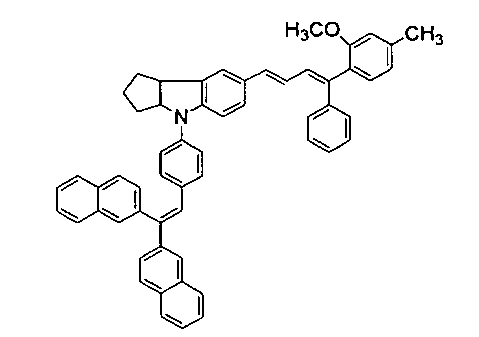

(Example compound 52, m=1, n=0)

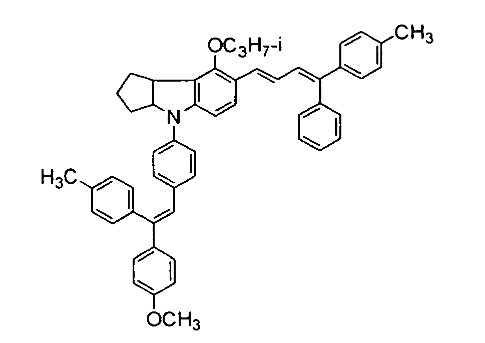

(Example compound 53, m=1, n=0)

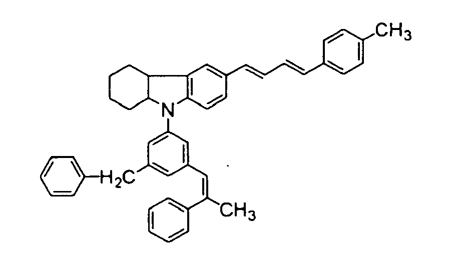

(Example compound 54, m=1, n=0)

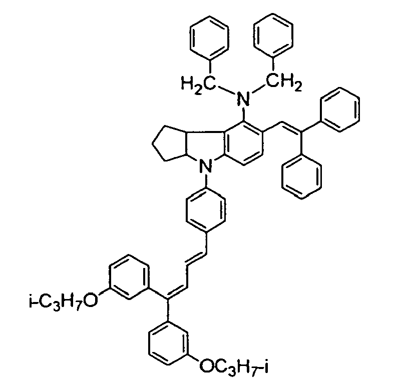

(Example compound 55, m=0, n=1)

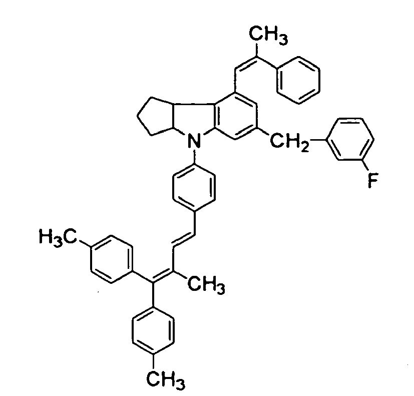

(Example compound 56, m=0, n=1)

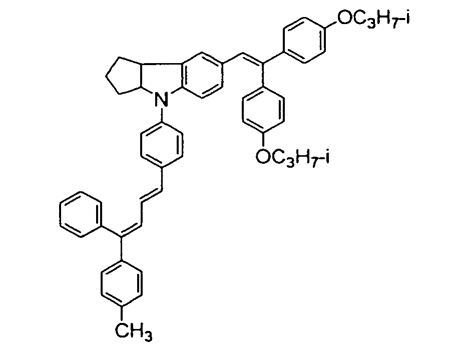

(Example compound 57, m=0, n=1)

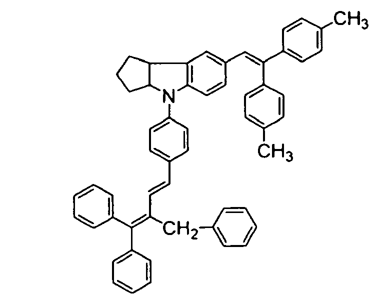

(Example compound 58, m=0, n=1)

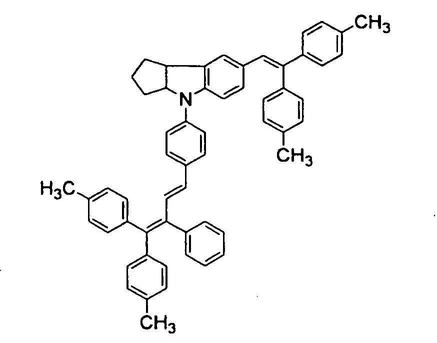

(Example compound 59, m=0, n=1)

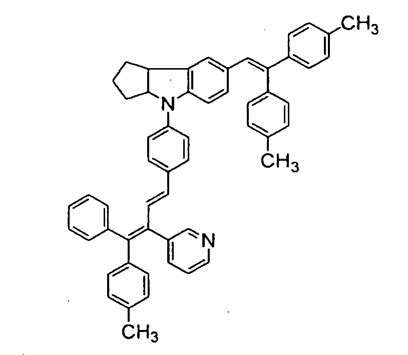

(Example compound 60, m=0, n=1)

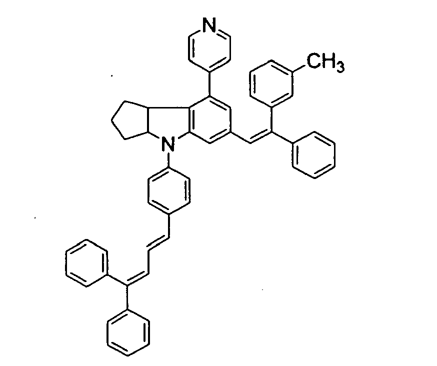

(Example compound 61, m=0, n=1)

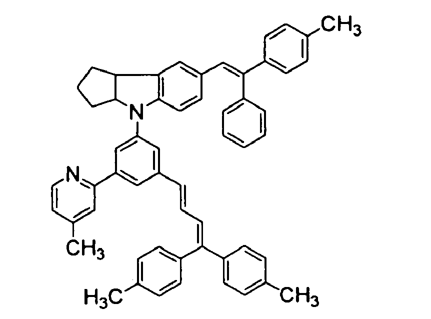

(Example compound 62, m=0, n=1)

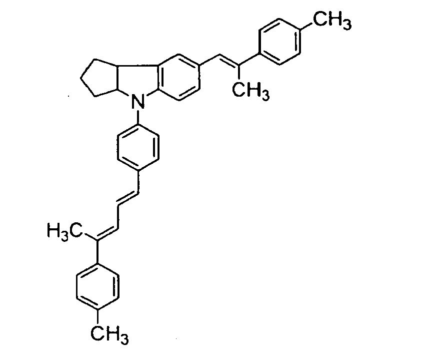

(Example compound 63, m=0, n=1)

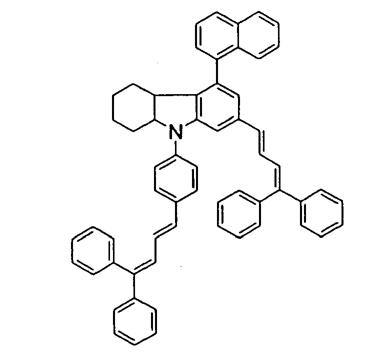

(Example compound 64, m=1, n=1)

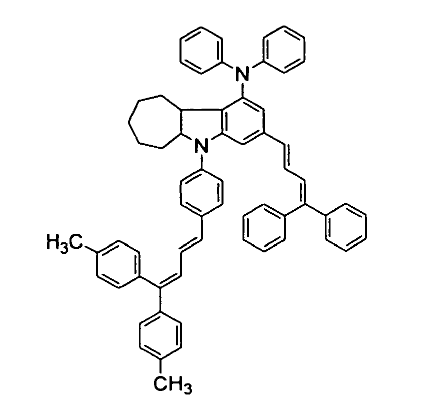

(Example compound 65, m=1, n=1)

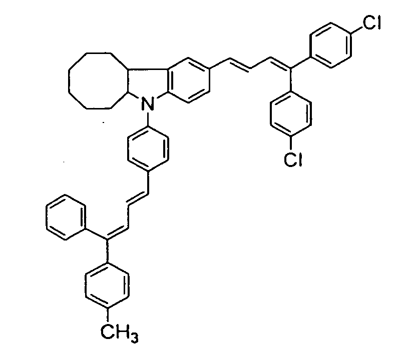

(Example compound 66, m=1, n=1)

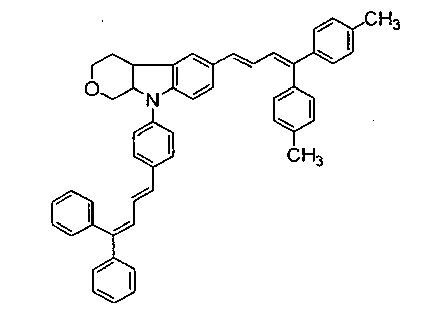

(Example compound 67, m=1, n=1)

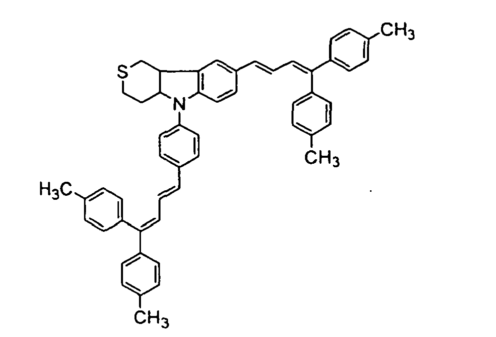

(Example compound 68, m=1, n=1)

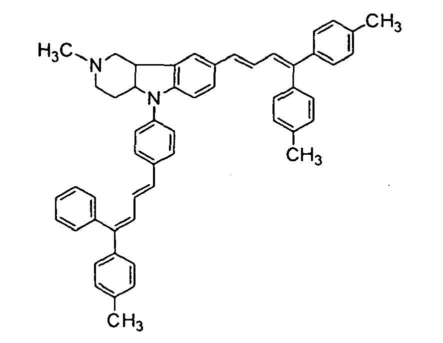

(Example compound 69, m=1, n=1)

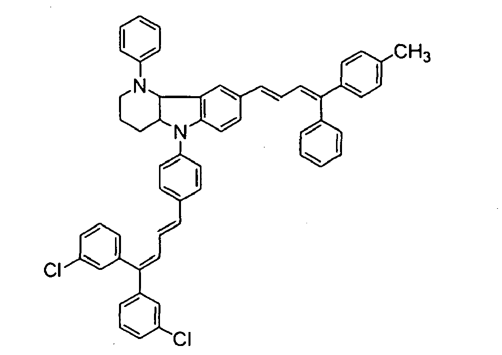

(Example compound 70, m=1, n=1)

In the present invention, among the indole derivatives exemplified above, an indole derivative of which the ring Z is a cyclopentane ring is preferred. Concretely, an indole derivative represented by the following formula (1'), 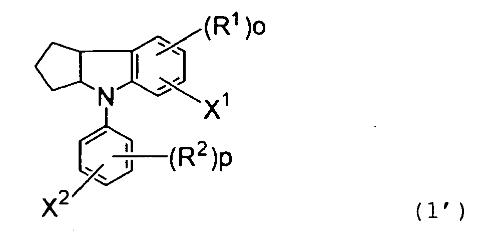 wherein,
R¹, R², X¹, X², k and j are as defined in the above general formula (1),
is preferred and, specifically, the one of which m is 0 in the general formula (1a) representing X¹ and of which n is 0 or 1 in the general formula (1b) representing X², is more preferred, and a compound having neither the group R¹ nor the group R² (k = j = 0) is most preferred.

### <Preparation of indole derivatives>

The indole derivative of the invention represented by the above general formula (1) can be synthesized by using an N-phenyl-substituted indole compound represented by the general formula (2) as a starting material. 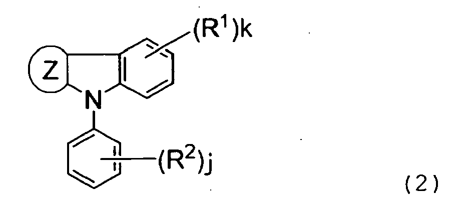 wherein R¹, R², ring Z, k and j are the same as those defined in the general formula (1).
The N-phenyl-substituted indole compound by itself is a known compound as has been disclosed in, for example, the above-mentioned patent document 15.

That is, the group X¹ in the general formula (1) is introduced into the above N-phenyl-substituted indole compound and, next, the group X² is introduced therein to produce the indole derivative of the present invention.

### (Introduction of the group X¹)

To introduce the group X¹ into the N-phenyl-substituted indole compound of the general formula (2), first, a carbonyl group is introduced into the benzene ring in the indole ring of the indole compound to obtain a carbonyl compound represented by the following general formula (3) or (4), 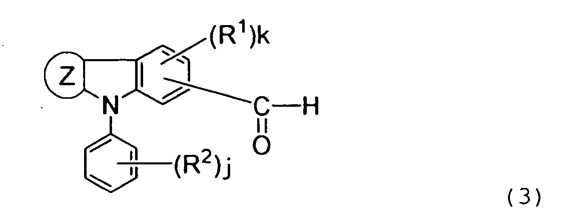 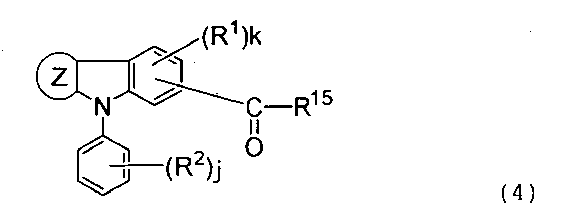 Wherein, in the general formula (3) or (4),
R¹, R², ring Z, k and j are as defined in the general formula (1), and
R¹⁵ is an alkyl group having 1 to 6 carbon atoms, an aromatic hydrocarbon group, an aromatic heterocyclic group or a condensed polycyclic aromatic group,
and, by utilizing the Wittig reaction, the introduced carbonyl group (formyl group or ketone group) is converted into the group X¹ represented by the general formula (1a).
In the above general formula (4), the group R¹⁵ corresponds to the group R³ or R⁵ (but excluding hydrogen atom) in the general formula (1a) that represents the group X¹.

To obtain the carbonyl compound of the above general formula (3) by introducing the carbonyl group (formyl group) into the N-phenyl-substituted indole compound, the indole compound may be reacted with a formylating agent such as N,N-dimethylformamide or N-methylformanilide in the presence of the phosphorous oxychloride.
The above reaction is usually carried out by using a solvent inert to the reaction, such as o-dichlorobenzene or benzene. Here, the above formylating agent may be used in excess amounts so as to also serve as a reaction solvent.

To obtain the carbonyl compound of the general formula (4) by introducing the carbonyl group (ketone group) into the N-phenyl-substituted indole compound, the indole compound may be reacted with an acid chloride (R¹⁵COCl) in the presence of a Lewis acid such as aluminum chloride, iron chloride or zinc chloride. The reaction is, usually, carried out by using a solvent inert to the reaction, such as nitrobenzene, dichloromethane or carbon tetrachloride.

To convert the carbonyl group in the carbonyl compound of the general formula (3) or (4) into the group X¹ by utilizing the Wittig reaction, further, the carbonyl compound may be reacted with a triphenylphosphine and a halogen compound represented by the following general formula (5), 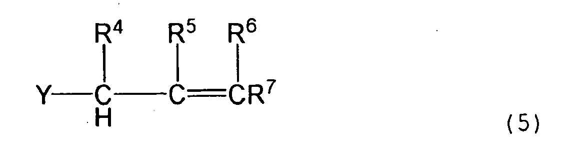 wherein
R⁴ to R⁷ are as defined in the above general formula (1a), and
Y is a halogen atom such as chlorine atom or bromine atom,
or by the following general formula (5'),

Y-CH (R⁶) (R⁷) (5')

wherein
R⁶ and R⁷ are as defined in the above general formula (1a), and
Y is a halogen atom such as chlorine atom or bromine atom.
By the above reaction, the group X¹ is introduced into the N-phenyl-substituted indole compound mentioned above. Namely, a compound represented by the following general formula (6) is obtained, 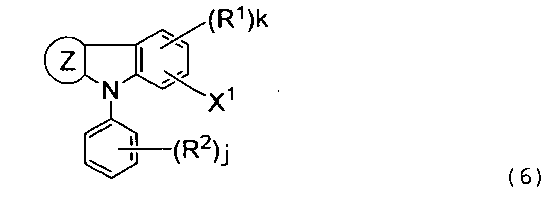 Wherein
R¹, R², ring Z, k, j and X¹ are as defined in the above general formula (1).

That is, if the halogen compound (or a corresponding Wittig reagent) of the general formula (5) is used, then the value of m of the group X¹ that is introduced is 1 and if the halogen compound (or a corresponding Wittig reagent) of the general formula (5') is used, then the value of m of the group X¹ that is introduced is 0.

Here, the above reaction (Wittig reaction) is conducted by using an organic solvent inert to the reaction, such as N,N-dimethylformamide, N,N-dimethylacetamide, tetrahydrofurane, dioxane, benzene or toluene.
Instead of using the above halogen compound and triphenylphosphine, it is also allowable to use a Wittig reagent (phosphoric acid ester) obtained by acting a trialkoxyphosphorus compound upon the halogen compound for the reaction with the carbonyl compound of the above general formula (3) or (4).

The reaction temperature in the Wittig reaction is preferably in a range of 10 to 200°C and, specifically, 20 to 100°C.
Further, the Wittig reaction is conducted preferably in the presence of a basic catalyst such as n-butyl lithium, phenyl lithium, sodium methoxide, sodium ethoxide or potassium tert-butoxide.

### (Introduction of the group X²)

Like introducing the group X¹, the group X² is introduced into the compound of the general formula (6) to which the group X¹ is introduced as described above; i.e., the carbonyl group (formyl group or ketone group) is introduced to form the carbonyl compound and, next, the carbonyl group is converted into the group X² by the Wittig reaction.

That is, in the same manner as described above, the formyl group or ketone group is introduced into the compound of the general formula (6) to obtain a compound represented by the following general formula (7), 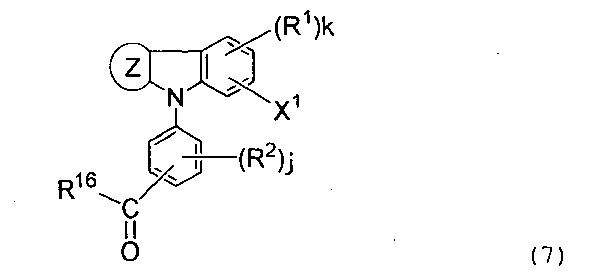 wherein
the ring Z, R¹, R², X¹, k and j are as defined above, and
R¹⁶ is a hydrogen atom, an alkyl group having 1 to 6 carbon atoms, an aromatic hydrocarbon group, an aromatic heterocyclic group or a condensed polycyclic aromatic group.
If formylation is executed in conducting the reaction, then R¹⁶ becomes a hydrogen atom. If ketone is obtained by using an acid chloride (R¹⁶COCl), then R¹⁶ becomes a group other than the hydrogen atom.
The above group R¹⁶ corresponds to R⁸ or R¹⁰ in the general formula (1b) that represents the group X².
Upon subjecting the carbonyl compound of the general formula (7) obtained as described above to the Wittig reaction in the same manner as when the group X¹ is introduced, the indole derivative represented by the general formula (1) of the present invention can be obtained.
In the Wittig reaction, a halogen compound or a Wittig reagent derived from the halogen compound represented by the following general formula (8) or (8'),

Y-CH(R⁹)-C(R¹⁰)=CR¹¹R¹² (8)

Y-CH(R¹¹) (R¹²) (8')

wherein
Y is a halogen atom such as chlorine atom or bromine atom, and
R⁹ to R¹² are as defined in the above general formula (1b),
is used instead of the halogen compound of the general formula (5) or (5') described above.
That is, if the halogen compound (or the corresponding Wittig reagent) of the general formula (8) is used, then the value of n of the group X² that is introduced is 1 and if the halogen compound (or the corresponding Wittig reagent) of the general formula (8') is used, then the value of n of the group X² that is introduced is 0.

In introducing the above group X¹ or the group X², the carbonyl group (formyl group) can also be introduced by introducing a halogen atom into the benzene ring by the halogenation reaction known per se., reacting it with magnesium or lithium to obtain an organometal compound thereof, and reacting the organometal compound with an N,N-dimethylformamide.
The above halogenation reaction has been described in detail in, for example, The Fourth Series of Experimental Chemistry No. 19, pp. 363-482, Nihon Kagakukai, 1992, and the reaction of the organometal compound with the dimethylformamide has been described in detail in, for example, The Fourth Series of Experimental Chemistry No. 21, pp. 23-44, pp. 179-196, Nihon Kagakukai, 1991.

After the group X¹ and the group X² are introduced into the starting indole compound as described above, refining is conducted by using column chromatography, activated carbon or activated clay. As required, further, the recrystallization or crystallization is conducted by using a solvent to obtain a desired indole derivative of the general formula (1).
The obtained compound can be identified by the IR measurement or the elemental analysis.

The thus obtained indole derivative of the invention has a high charge mobility and is favorably used as a charge transporting agent for an organic photosensitive material for electrophotography. Further, the indole derivative of the invention represented by the general formula (1) can also be used as a material for an organic electroluminescence (EL) element.

### <Organic photosensitive material for electrophotography>

The organic photosensitive material using the indole derivative of the invention as a charge transporting agent comprises an electrically conducting substrate on which is formed a photosensitive layer that contains the charge transporting agent as well as a charge generating agent. Here, the photosensitive layer includes the two types, i.e., the one type is of a single layer containing the charge transporting agent and the charge generating agent (single layer type photosensitive layer), and the another type comprises a charge transporting layer containing the charge transporting agent and a charge generating layer containing the charge generating agent (lamination type photosensitive layer).

As the electrically conducting substrate for supporting the photosensitive layer, there can be used an electrically conducting material that has been used for the known photosensitive materials for electrophotography. Concretely, there can be used a sheet of a metal such as copper, aluminum, silver, iron, zinc or nickel, or an alloy thereof, or the sheet in the form of a drum. Or there can be used a plastic film or a plastic cylinder on which the above metals are vapor-deposited or electroplated, or a glass, a paper or a plastic film on which a layer of an electrically conducting compound such as an electrically conducting polymer, indium oxide or tin oxide is applied or deposited.

The photosensitive layer can be formed on the electrically conducting substrate by vapor deposition depending upon the type of the photosensitive layer (in the case of the lamination type photosensitive layer) but is, usually, formed by using a resin binder. That is, the charge transporting agent and the charge generating agent are dissolved together with the resin binder in an organic solvent to prepare a coating solution which is then applied onto the electrically conducting substrate and is dried to thereby form a photosensitive layer of the single layer type or the lamination type.

As the resin binder for forming the photosensitive layer, there can be used a thermoplastic or thermosetting resin that has heretofore been used for forming photosensitive layers. Concrete examples include (meth)acrylic resins such as polyacrylate and polymethacrylate, as well as polyamide resin, acrylonitrile resin, vinyl chloride resin, acetal resin, butylal resin, vinyl acetate resin, polystylene resin, polyolefin resin, cellulose ester, phenol resin, epoxy resin, polyester, alkyd resin, silicone resin, polycarbonate resin, polyurethane resin and polyimide resin. In addition to the above, there can be, further, used an organic photoconductive polymer such as polyvinylcarbazole, polyvinylanthracene or polyvinylpyrene as a resin binder.
The above resin binder can be used in one kind or in two or more kinds in combination. As the binder resin for the charge transporting layer of the lamination type photosensitive layer of the invention, in particular, the polycarbonate resin is preferably used. Specifically, the polycarbonate resin having a recurring unit represented by the following formula (A) is preferred.

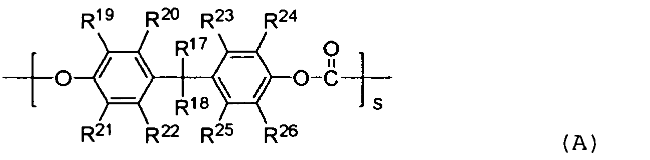

wherein,
R¹⁷ and R¹⁸ may be same or different, and are hydrogen atoms, straight-chain or branched alkyl groups having 1 to 4 carbon atoms, straight-chain or branched alkoxy groups having 1 to 4 carbon atoms, or phenyl groups which may be substituted with halogen atoms, and together may form a ring,
R¹⁹ to R²⁶ may be same or different, and are hydrogen atoms, halogen atoms, straight-chain or branched alkyl groups having 1 to 6 carbon atoms, straight-chain or branched alkoxy groups having 1 to 6 carbon atoms, or substituted or unsubstituted phenyl groups, and s is a positive integer.

Among the polycarbonate resins having the recurring unit represented by the above formula (A), the following polycarbonate resins are preferred examples.
(1) A bisphenol A type polycarbonate resin having a recurring unit represented by the following formula (B) (e.g., Iupilon E Series manufactured by Mitsubishi Gas Kagaku Co.): 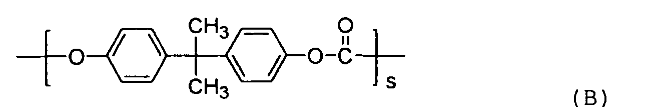 wherein
   s is a positive integer.
(2) A bisphenol Z type polycarbonate resin having a recurring unit represented by the following formula (C) (e.g., Iupilon Z Series manufactured by Mitsubishi Gas Kagaku Co.): 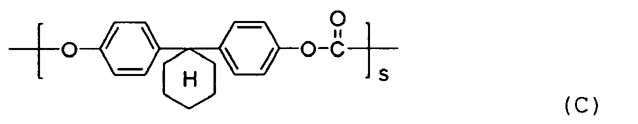 wherein
   s is a positive integer.
(3) A copolymerized polycarbonate resin containing bisphenol A, bisphenol Z or biphenol as a structural unit (see, for example, JP-A-09-179961).

As the copolymerized polycarbonate resin of (3) above, there can be concretely exemplified a bisphenol/biphenol type polycarbonate resin represented by the following formula (D): 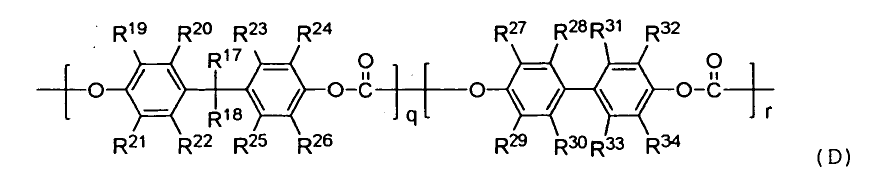 wherein,
R¹⁷ to R²⁶ are as defined in the above formula (A),
R²⁷ to R³⁴ may be same or different, and are hydrogen atoms, halogen atoms, straight-chain or branched alkyl groups having 1 to 6 carbon atoms, straight-chain or branched alkoxy groups having 1 to 6 carbon atoms, or substituted or unsubstituted phenyl groups,
R²⁷ and R²⁸, R²⁹ and R³⁰, R³¹ and R³², and R³³ and R³⁴ together may form rings, respectively, and
q and r represent mol numbers of the recurring units and, preferably, are numbers satisfying q/(q + r) = 0.1 to 0.9.
More concretely, there can be exemplified a bisphenol A/biphenol type polycarbonate resin represented by the following formula (E): 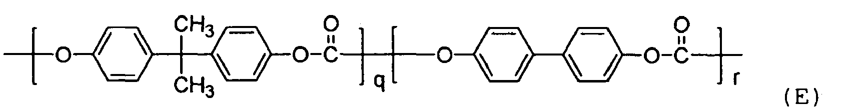 wherein
q and r represent mol numbers of the recurring units, and the ratio of q/(q + r) is 0.85.

In addition to the polycarbonate resin having the recurring unit of the above formula (A), polycarbonate resins having recurring units of the following formulas (F) to (I), too, can be preferably used.

(4) A polycarbonate resin having a recurring unit represented by the following formula (F)(e.g., see JP-A-6-214412): 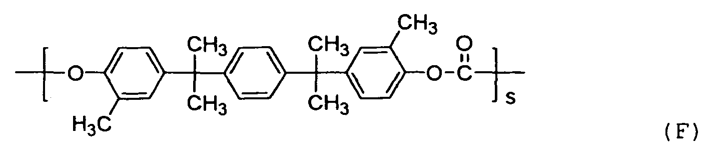 wherein
   s is a positive integer.
(5) A polycarbonate resin having a recurring unit represented by the following formula (G)(e.g., see JP-A-6-222581): 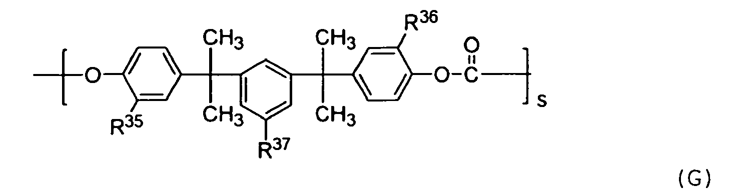 wherein,
   R³⁵ to R³⁷ may be same or different, and are hydrogen atoms, halogen atoms, straight-chain or branched alkyl groups having 1 to 6 carbon atoms, cycloalkyl groups, substituted or unsubstituted aromatic hydrocarbon groups, substituted or unsubstituted condensed polycyclic aromatic groups or alkyl groups substituted with aromatic hydrocarbon groups or condensed polycyclic aromatic groups, and
   s is a positive integer.
(6) A siloxane type polycarbonate resin having a recurring unit represented by the following formula (H)(see, for example, JP-A-5-088398, JP-A-11-065136): 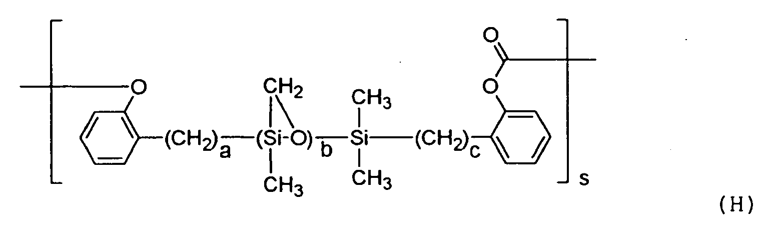 wherein
   a, b, c and s are positive integers,
(7) or represented by the following formula (I): 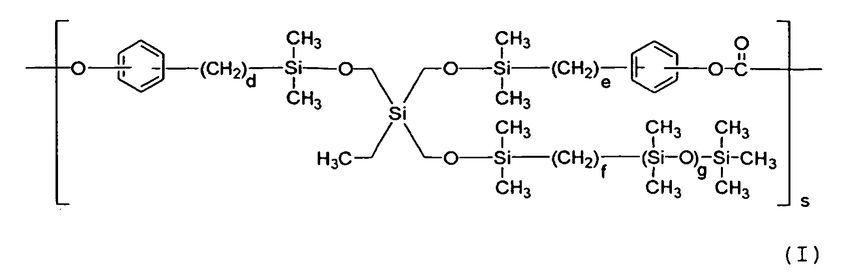 wherein
   d, e, f, g and s are positive integers.

There is no particular limitation on the organic solvent used for preparing a coating solution that is used for forming the photosensitive layer provided it is capable of dissolving the charge transporting agent (e.g., indole derivative of the general formula (1)) or the resin binder blended therein and is, further, capable of dissolving or dispersing the charge generating agent. Usually, however, the following compounds are used alone or in a combination of two or more kinds.
Alcohols such as methanol, ethanol and 2-propanol;

Ketones such as acetone, methyl ethyl ketone and cyclohexanone;
Amides such as N,N-dimethylformamide and N,N-dimethylacetamide;
Sulfoxides such as dimethyl sulfoxide, etc.;
Ethers such as tetrahydrofurane, dioxane, dioxolane, ethylene glycol dimethyl ether, diethyl ether, diisopropyl ether and tert-butylmethyl ether;
Esters such as ethyl acetate and methyl acetate;
Aliphatic halogenated hydrocarbons such as methylene chloride, chloroform, 1,2-dichloroethane, dichloroethylene, carbon tetrachloride and trichloroethylene;
Aromatic halogenated hydrocarbons such as chlorobenzene and dichlorobenzene;
Aromatic hydrocarbons such as benzene, toluene and xylene; and
Aliphatic hydrocarbons such as pentane, hexane, heptane, octane and cyclohexane.

The coating solution using the above organic solvent is prepared by dissolving or dispersing the resin binder as well as the charge transporting agent and the charge generating agent in the organic solvent depending upon the form of the photosensitive layer that is to be formed.
Namely, when the photosensitive layer of the single layer type is to be formed, the coating solution is prepared by adding the charge transporting agent, charge generating agent and resin binder into the organic solvent.
When the photosensitive layer of the lamination type is to be formed, there are prepared the coating solution for forming the charge transporting layer by adding the charge transporting agent and resin binder into the organic solvent, and the coating solution for forming the charge generating layer by adding the charge generating agent and resin binder into the organic solvent.

In order to improve the stability and applicability of the coating solution and to improve the charge properties and durability of the photosensitive layer, further, the coating solutions may contain various additives as required.
As the additives, there can be exemplified plasticizers such as biphenylene type compound, m-terphenyl compound and dibutyl phthalate; surface lubricating agents such as silicone oil, graft-type silicone polymer and various fluorocarbons; potential stabilizers such as dicyanovinyl compound and carbazole derivative; monophenol type antioxidants such as 2,6-di-tert-butyl-4-methylphenol; bisphenol type antioxidant; amine type antioxidants such as 4-diazabicyclo[2,2,2]octane, etc.; salicylic acid type antioxidant; antioxidants such as tocophenol, etc.; ultraviolet ray absorber; and sensitizer. These additives are suitably used in amounts in a range in which they do not impair the properties of the photosensitive layer or the applicability of the coating solution.

The above coating solution can be applied by a known method such as dip coating method, spray coating method, spinner coating method, Meyer bar coating method, blade coating method, roller coating method or curtain coating method.

A desired photosensitive layer is formed by drying the coating of the coating solution. In the case of the lamination type photosensitive layer, the charge generating layer or the charge transporting layer is formed on the electrically conducting substrate and, thereafter, the charge transporting layer or the charge generating layer is formed thereon.
The above drying is conducted, desirably, by holding the coating at room temperature followed by heating. The heating is conducted, desirably, at a temperature of 30 to 200°C for 5 minutes to 2 hours by blowing or without blowing the air.

Prior to forming the photosensitive layer, an underlying layer may be formed on the electrically conducting substrate and the photosensitive layer may be formed on the underlying layer. The underlying layer is for imparting a barrier function for preventing the deterioration of the surface of the electrically conducting substrate or for improving the adhesion between the photosensitive layer and the surface of the electrically conducting substrate. The underlying layer can be formed by a thin layer of a resin such as polyvinyl alcohol; nitrocellulose; casein; ethylene/acrylic acid copolymer; polyamide such as nylon; polyurethane; or gelatin;, an aluminum oxide layer or a resin layer in which a metal oxide such as titanium oxide is dispersed.
It is desired that the underlying layer has a thickness in a range of 0.1 to 5 µm and, specifically, in a range of 0.5 to 3 µm. If the underlying layer has a too large thickness, inconvenience occurs such as an increase in the residual potential of the photosensitive material due to a rise in the resistivity.

On the photosensitive layer formed as described above, it is, further, allowable to suitably form a protection layer for preventing the photosensitive layer from being deteriorated by ozone or nitrogen oxides or for preventing the photosensitive layer from being abraded.

In the present invention as described above, an indole derivative of the general formula (1) is used as a charge transporting agent in the photosensitive layer that is formed as described above. The amount of use of the indole derivative may differ depending upon the kind of the photosensitive layer that is formed but is, usually, in a range of 10 to 1000 parts by weight, preferably, 30 to 500 parts by weight and, more preferably, 40 to 200 parts by weight per 100 parts by weight of the resin binder, the indole derivative preferably being present in the single layer type photosensitive layer or the charge transporting layer of the lamination type photosensitive layer.

The above photosensitive layer may further contain, as required, charge transporting agents other than the above indole derivative in an amount in a range in which they do not impair excellent properties of the indole derivative.

The other charge transporting agents can be represented by, for example, the following compounds.
Fluorenone type compounds such as chloranil, tetracyanoethylene and 2,4,7-trinitro-9-fluorenone;
Nitro compounds such as 2,4,8-trinitrothioxanthone and dinitroanthracene;
Hydrazone type compounds such as N,N-diethylaminobenzaldehyde, N,N-diphenylhydrozone, N-methyl-3-carbazolylaldehyde, and N,N-diphenylhydrazone;
Oxadiazole type compounds such as 2,5-di(4-dimethylaminophenyl)-1,3,4-oxadiazole, etc.;
Styryl type compounds such as 9-(4-diethylaminostyryl)anthracene, etc.;
Carbazole type compounds such as poly-N-vinylcarbazole and N-ethylcarbazole;
Pyrazoline type compounds such as 1-phenyl-3-(p-dimethylaminophenyl)pyrazoline, etc.;
Oxazole type compounds such as 2-(p-diethylaminophenyl)-4-(p-dimethylaminophenyl)-5-(2-chlorophenyl)oxazole, etc.;
Isooxazole type compound;
Thiazole type compounds such as 2-(p-diethylaminostyryl)-6-diethylaminobenzothiazole, etc.;
Amine type compounds such as triphenylamine and 4,4'-bis[N-(3-methylphenyl)-N-phenylamino]diphenyl; etc.; and
Stilbene type compounds such as α-phenylstilbene, etc.

In addition to the above, there can be, further, used thiadiazole type compound, imidazole type compound, pyrazole type compound, indole type compound other than the one of the general formula (1), triazole type compound, tetraphenylbutadiene type compound or triphenylmethane type compound as a charge transporting agent in combination with the indole derivative of the general formula (1).

It is further allowable to use, in combination with the indole derivative of the general formula (1), a hydrazone compound represented by the following general formula (9): 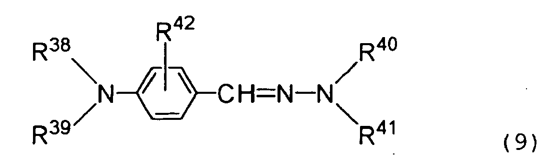 wherein,
R³⁸ and R³⁹ may be same or different, and are straight-chain or branched lower alkyl groups having 1 to 4 carbon atoms, substituted or unsubstituted aromatic hydrocarbon groups, substituted or unsubstituted condensed polycyclic aromatic groups, or substituted or unsubstituted aralkyl groups,
R⁴⁰ and R⁴¹ may be same or different, and are straight-chain or branched lower alkyl groups which have 1 to 4 carbon atoms and which may have a substituent, substituted or unsubstituted aromatic hydrocarbon groups, substituted or unsubstituted condensed polycyclic aromatic groups, substituted or unsubstituted aralkyl groups, or substituted or unsubstituted heterocyclic groups, and R⁴⁰ and R⁴¹ together may form a ring,
R⁴² is a hydrogen atom, a straight-chain or branched lower alkyl group having 1 to 4 carbon atoms, a substituted or unsubstituted aromatic hydrocarbon group, a substituted or unsubstituted condensed polycyclic aromatic group, a substituted or unsubstituted aralkyl group, a straight-chain or branched lower alkoxy group having 1 to 4 carbon atoms, or a halogen atom, and R⁴² and R³⁸ or R³⁹ together may form a ring,
(see, for example, JP-B-55-042380, JP-A-60-340999, JP-A-61-023154), a triphenylamine dimer represented by the following general formula (10): 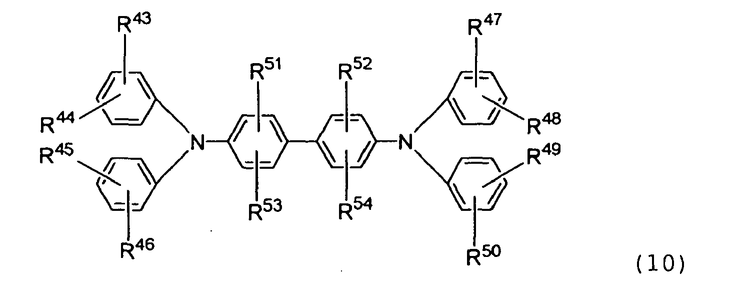 wherein,
R⁴³ to R⁵⁴ may be same or different, and are hydrogen atoms, straight-chain or branched lower alkyl groups having 1 to 4 carbon atoms, straight-chain or branched lower alkoxy groups having 1 to 4 carbon atoms, straight-chain or branched lower alkyl groups substituted with a halogen atom and having 1 to 4 carbon atoms, straight-chain or branched lower alkoxy groups substituted with a halogen atom and having 1 to 4 carbon atoms, substituted or unsubstituted aromatic hydrocarbon groups, substituted or unsubstituted condensed polycyclic aromatic groups, or halogen atoms,
(see, for example, JP-B-58-032372), or a distyryl type compound represented by the following general formula (11): 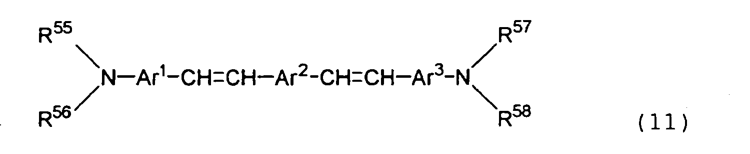 wherein,
R⁵⁵ to R⁵⁸ may be same or different, and are straight-chain or branched lower alkyl groups having 1 to 4 carbon atoms, substituted or unsubstituted aromatic hydrocarbon groups, or substituted or unsubstituted condensed polycyclic aromatic groups,
Ar¹ and Ar³ may be same or different, and are substituted or unsubstituted phenylene groups, and
Ar² is a diavalent group of a substituted or unsubstituted monocyclic or polycyclic aromatic hydrocarbon having 4 to 14 carbon atoms, or a divalent group of a substituted or unsubstituted heterocyclic ring, and wherein,
Ar¹, Ar² and Ar³, when having substituents, are substituted by one or two or more substituents selected from the straight-chain or branched lower alkyl groups having 1 to 4 carbon atoms, straight-chain or branched lower alkoxy groups having 1 to 4 carbon atoms, substituted or unsubstituted aryloxy groups and halogen atoms,
(see, for example, U.S. Patent No. 3873312).

The charge generating agents contained in the photosensitive layer are the materials that absorb light and generate electric charge at high efficiencies, and can be roughly grouped into the inorganic charge generating agents and the organic charge generating agents.
As the inorganic charge generating agents, there have been known selenium, selenium-tellurium and amorphous silicon.
As the organic charge generating agents, there have been known cationic dyes (e.g., thiapyrylium salt type dye, azulenium salt type dye, thiacyanine type dye, quinocyanine type dye), squalium salt type pigment, phthalocyanine type pigment, polycyclic quinone pigments (e.g., anthanthrone type pigment, dibenzpyrenequinone type pigment, pyranthrone type pigment), indigo type pigment, quinacrydone type pigment, azo pigment, pyrrolopyrrole type pigment and perylene type pigment.
In the present invention, either the inorganic charge generating agents or the organic charge generating agents can be used in one kind or in two or more kinds in combination and, specifically, the organic charge generating agents are preferred.

Among the organic charge generating agents, specifically preferred are phthalocyanine type pigment, azo pigment, perylene type pigment and polycyclic quinone pigment. Concrete examples are as described below.

Concrete examples of the phthalocyanine type pigment include alkoxytitanium phthalocyanine (Ti(OR)₂Pc), oxotitanium phthalocyanine (TiOPc), copper phthalocyanine (CuPc), metal-free phthalocyanine (H₂Pc), hydroxygallium phthalocyanine (HOGaPc), vanadyl phthalocyanine (VOPc) and chloroindium phthalocyanine (ClInPc). More closely, as the TiOPc, there can be exemplified α-type TiOPc, β-type TiOPc, γ -type TiOPc, m-type TiOPc, Y-type TiOPc, A-type TiOPc, B-type TiOPc and TiOPc amorphous. As the H₂Pc, there can be exemplified α-type H₂Pc, β -type H₂Pc, τ-type H₂Pc and χ-type H₂Pc.

As the azo pigment, there can be exemplified a monoazo compound, a bisazo compound and a trisazo compound and, particularly preferably, the bisazo compounds represented by the following structural formulas (J) to (L) and the trisazo compound represented by the following structural formula (M). Structural formula (J): 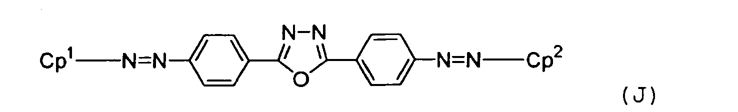 wherein Cp¹ and Cp² may be same or different, and are the groups represented by the following structural formula (12) or the following structural formula (13), 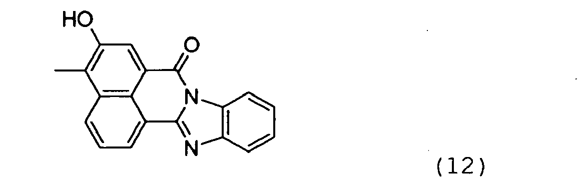 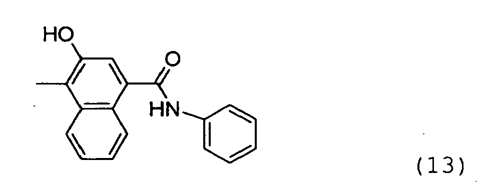 Structural formula (K): 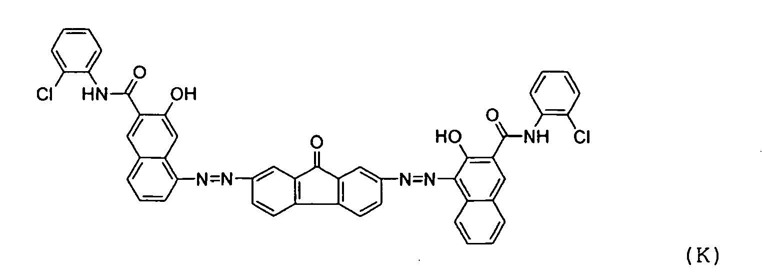 Structural formula (L): 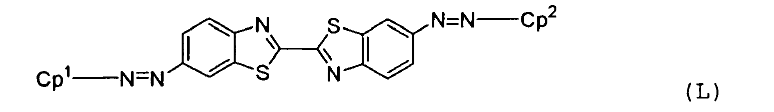 wherein Cp¹ and Cp² may be same or different, and are the groups represented by the above structural formula (12) or (13).
Structural formula (M): 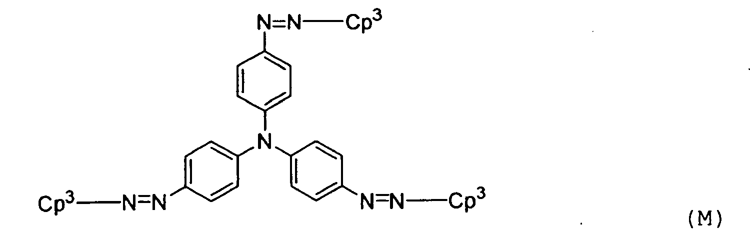 wherein Cp³ is a group represented by the following structural formula (14),

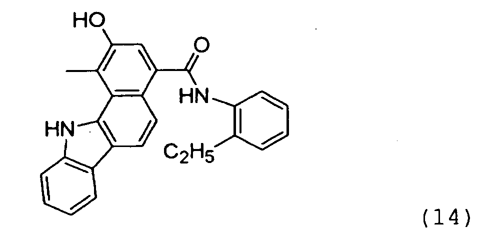

As the perylene type compound or the polycyclic quinone type pigment, concretely, the compounds represented by the following structural formulas (N) and (O) are particularly preferred.
Structural formula (N): 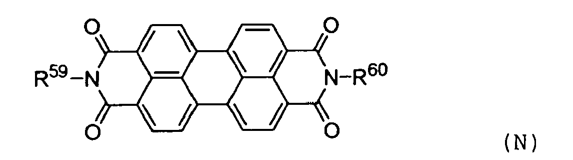 wherein R⁵⁹ and R⁶⁰ may be same or different, and are straight-chain or branched lower alkyl groups having 1 to 4 carbon atoms, substituted or unsubstituted aromatic hydrocarbon groups, or substituted or unsubstituted condensed polycyclic aromatic groups,
Structural formula (O):

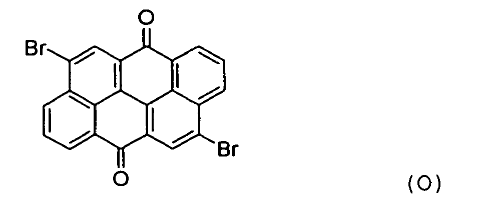

The ratio of the charge generating agent that occupies the photosensitive layer may differ depending upon the type of the photosensitive layer. In the case of the single layer type photosensitive layer, the amount of the charge generating agent is, usually, 0.2 to 40 parts by mass and, specifically, 0.5 to 20 parts by mass per 100 parts by mass of the resin binder. In the charge generating layer in the lamination type photosensitive layer, the amount of the charge generating agent is 30 to 400 parts by mass and, specifically, 60 to 300 parts by mass per 100 parts by mass of the resin binder.

In the case of the single layer type photosensitive layer, the thickness of the photosensitive layer is about 5 to about 100 µ m and, specifically, about 15 to about 45 µ m.
In the case of the lamination type photosensitive layer, the thickness of the charge generating layer is preferably about 0.01 to about 5 µ m and, specifically, about 0.05 to about 2 µ m, and the thickness of the charge transporting layer is preferably about 5 to about 40 µ m and, specifically, about 10 to about 30 µ m.

Upon being electrically connected to the charge generating layer, the charge transporting layer in the lamination type photosensitive layer is allowed to receive the charge carrier injected from the charge generating layer in the presence of an electric field and can possess a function for transporting the charge carrier to the surface of the photosensitive layer. Here, the charge transporting layer may be laminated on the charge generating layer or may be laminated thereunder. From the standpoint of suppressing the deterioration of the charge generating layer, however, it is desired that the charge transporting layer is laminated on the charge generating layer.

Owing to excellent properties of the indole derivative of the above general formula (1), the organic photosensitive material for electrophotography having the photosensitive layer containing the indole derivative as a charge transporting agent, effectively avoids the precipitation of crystals or the occurrence of pinholes at the time of forming the photosensitive layer, and features a high degree of sensitivity and a low residual potential. Even after having repetitively formed the images by electrophotography, therefore, the organic photosensitive material makes it possible to form vivid images for extended periods of time.
By using the organic photosensitive material, the image by electrophotography is formed through a process of charging the surface of the photosensitive material to a predetermined polarity by using, for example, a corona charger, forming an electrostatic latent image by the irradiation with light (exposing the image to light) based on the image data, developing the electrostatic latent image by using a known developing agent to form a toner image on the surface of the photosensitive material, transferring the toner image onto a predetermined recording material, and fixing the transferred toner image on the recording material by heat and pressure. After the toner image has been transferred, the electric charge is removed from the surface of the photosensitive material by the irradiation with light for removing charge, and the toner that is remaining is removed by using a cleaning blade or the like to be ready for the next imaging process.

### EXAMPLES

The invention will be concretely described below by way of Examples which, however, are in no way to limit the invention.

### [Synthesis Example 1 (synthesis of an example compound 4)]

An N-phenyl-substituted indole compound represented by the following formula (15) was prepared as a starting material. This compound was a known compound disclosed in the patent document 16. 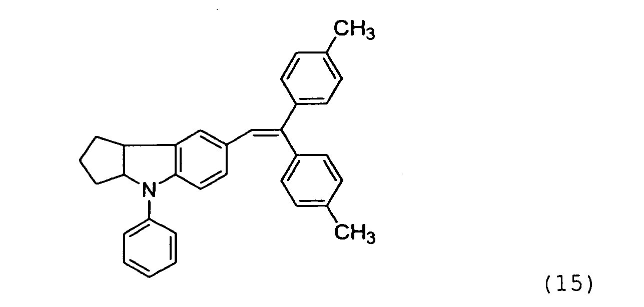 Into a reaction container were introduced 15 g of the above N-phenyl-substituted indole compound, 4.5 g of an N, N-dimethylformamide and 8 g of toluene, and to which was added 9 g of a phosphoryl trichloride dropwise. While heating, the mixture was stirred at 80°C for 3 hours. After left to cool, 8 g of water was added thereto dropwise while being cooled, followed by the addition of sodium carbonate to render the reaction solution to be alkaline.
Next, the solution was heated at 60°C for 3 hours and was, thereafter, extracted with toluene. The extract was washed with water and next with saturated brine and was, thereafter, dried on magnesium sulfate. Upon distilling off the solvent, there was obtained 14.4 g of a yellow solid formyl compound represented by the following structural formula (16).

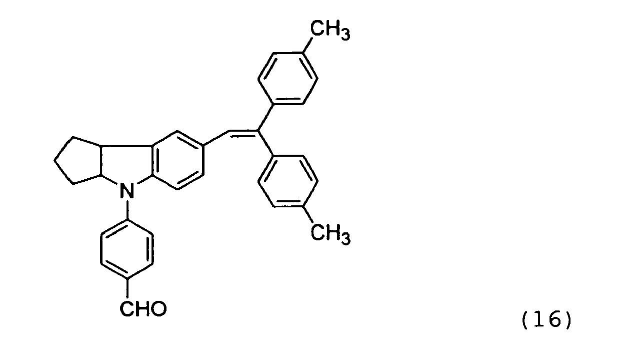

4 Grams of the obtained formyl compound and 9.6 g of a diphenylmethyl phosphorous acid diethyl ester were dissolved in 50 ml of an N,N-dimethylformamide, and to which was added 1.7 g of a sodium methylate while maintaining the temperature at 20 ± 5°C. After stirred for 2 hours, 30 ml of ion-exchanged water was added thereto, and the mixture was refined in a customary manner to obtain 3.1 g of a compound represented by the following formula (17)(yield, 56%). 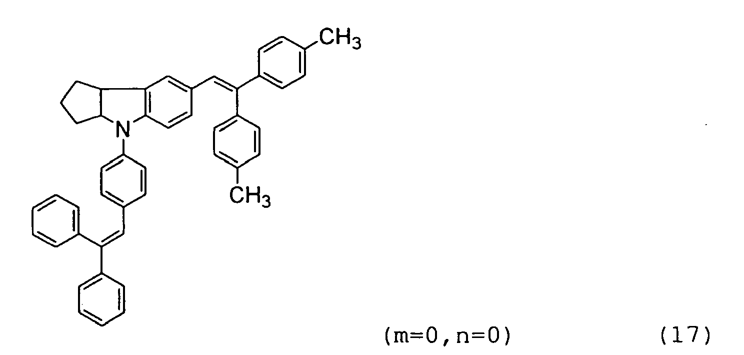 This compound was a yellow solid and corresponded to the above example compound 4.

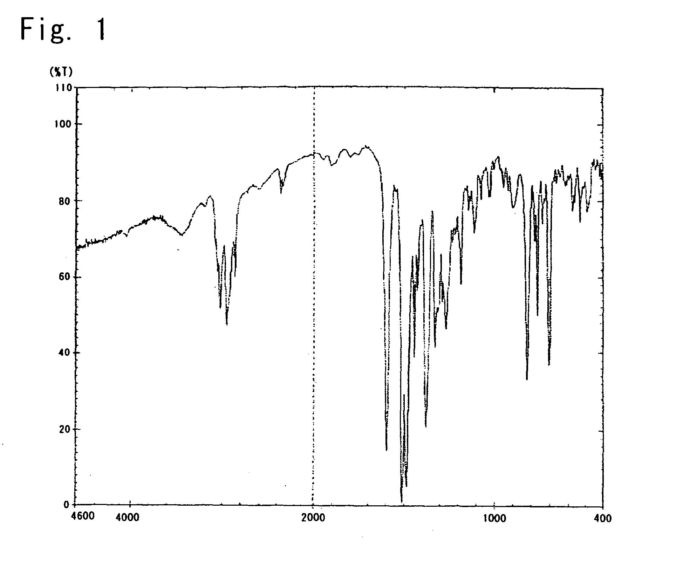

Through the elemental analysis and IR measurement, it was confirmed that the above yellow solid was a compound represented by the above formula (17) . The IR spectrum thereof was as shown in Fig. 1.

Values of the elemental analysis were as follows:

| | Carbon | Hydrogen | Nitrogen |
|---|---|---|---|
| Measured (%) | 91.10 | 6.69 | 2.21 |
| Calculated (%) | 91.07 | 6.67 | 2.26 |

### [Synthesis Example 2 (Synthesis of an example compound 5)]

4 Grams of the formyl compound of the above structural formula (16) obtained in Synthesis Example 1 and 10.5 g of a ditolylmethyl phosphorous acid diethyl ester were dissolved in 50 ml of the N,N-dimethylformamide, and to which was added 1.7 g of the sodium methylate while maintaining the temperature at 20 ± 5°C. After stirred for 2 hours, 30 ml of ion-exchanged water was added thereto, and the mixture was refined in a customary manner to obtain 2.8 g of a compound represented by the following formula (18)(yield, 51%). 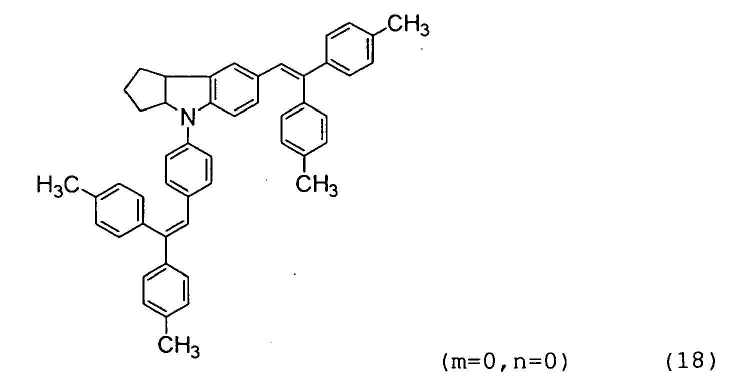 This compound was a yellow solid and corresponded to the above example compound 5.

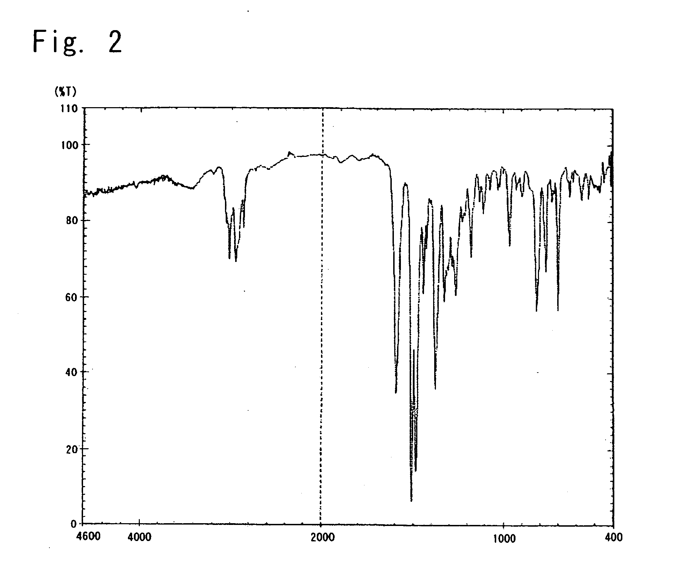

Through the elemental analysis and IR measurement, it was confirmed that the above yellow solid was a compound represented by the above formula (18). The IR spectrum thereof was as shown in Fig. 2.

Values of the elemental analysis were as follows:

| | Carbon | Hydrogen | Nitrogen |
|---|---|---|---|
| Measured (%) | 90.89 | 7.02 | 2.09 |
| Calculated (%) | 90.84 | 7.00 | 2.16 |

### [Synthesis Example 3 (Synthesis of an example compound 22)]

4 Grams of the formyl compound of the above structural formula (16) obtained in Example 1 and 10.4 g of a diphenylpropylene phosphorus acid diethyl ester were dissolved in 50 ml of the N,N-dimethylformamide, and to which was added 1.8 g of the sodium methylate while maintaining the temperature at 20 ± 5°C. After stirred for 2 hours, 30 ml of ion-exchanged water was added thereto, and the mixture was refined in a customary manner to obtain 3.3 g of a compound represented by the following formula (19)(yield, 60%). 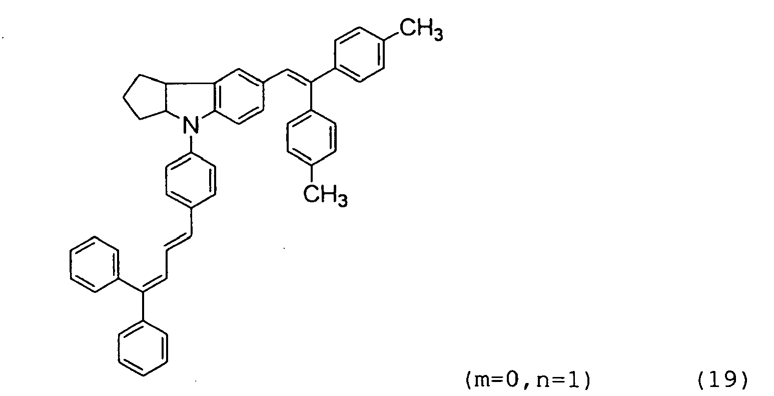 This compound was a yellow solid and corresponded to the above example compound 22.

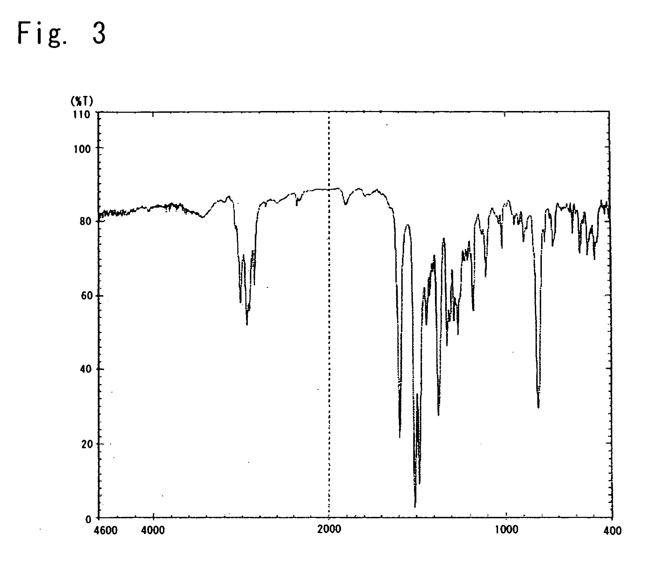

Through the elemental analysis and IR measurement, it was confirmed that the above yellow solid was a compound represented by the above formula (19). The IR spectrum thereof was as shown in Fig. 3.

Values of the elemental analysis were as follows:

| | Carbon | Hydrogen | Nitrogen |
|---|---|---|---|
| Measured (%) | 91.15 | 6.74 | 2.11 |
| Calculated (%) | 91.12 | 6.71 | 2.17 |

### [Photosensitive Material Example 1]

One part by mass of an alcohol-soluble polyamide (Amilan CM-4000 manufactured by Toray Co.) was dissolved in 13 parts by mass of methanol. 5 Parts by mass of titanium oxide (TIPAQUE CR-EL manufactured by Ishihara Sangyo Co.) was added thereto and was dispersed therein for 8 hours by using a paint shaker to prepare a coating solution for undercoating.
Next, by using a wire bar, the coating solution was applied onto the aluminum surface of an aluminum-deposited PET film (electrically conducting substrate) and was dried under normal pressure at 60°C for one hour to form an undercoating of a thickness of 1 µ m.

The following titanylphthalocyanine was provided as a charge generating agent. 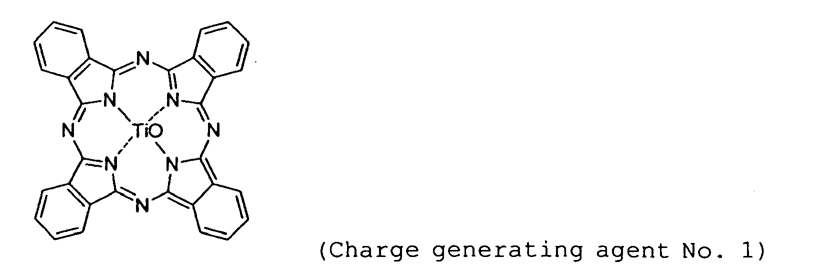 The titanylphthalocyanine exhibited intense peaks at diffraction angles 2 θ ± 2° of 9.6, 24.1 and 27.2 in the X-ray diffraction spectrum of Cu-Kα.

A polyvinyl butyral resin (S-LEC BL-S manufactured by Sekisui Kagaku Kogyo Co.) was provided as a resin binder for the charge generating layer.

1.5 Parts by mass of the above titanylphthalocyanine (charge generating agent No. 1) was added to 50 parts by mass of a cyclohexanone solution containing 3% of the polyvinyl butyral resin and was dispersed therein for one hour by using an ultrasonic wave dispersing machine. The obtained dispersion solution was applied onto the undercoating by using the wire bar, and was dried under normal pressure at 110°C for one hour to form a charge generating layer of a thickness of 0.6 µ m.

On the other hand, the indole derivative (example compound 4) of the formula (17) obtained in Synthesis Example 1 was provided as a charge transporting agent.
Further, a polycarbonate resin (Iupilon Z manufactured by Mitsubishi Engineering Plastic Co.) was provided as a binder resin for the charge transporting layer.

1.5 Parts by mass of the above charge transporting agent was added to 18.75 parts by mass of a dichloroethane solution containing 8.0% of the polycarbonate resin, and the charge transporting agent (indole derivative of the formula (17)) was completely dissolved therein by applying ultrasonic waves. The solution was applied onto the charge generating layer by using the wire bar and was dried under normal pressure at 110°C for 30 minutes to form a charge transporting layer of a thickness of 20 µ m to thereby prepare a laminated photosensitive material No. 1.

### [Photosensitive Material Example 2]

A laminated photosensitive material No. 2 was prepared in the same manner as in the photosensitive material Example 1 but using the indole derivative (example compound 5) of the formula (18) obtained in Synthesis Example 2 instead of using the charge transporting agent used in the photosensitive material Example 1.

### [Photosensitive Material Example 3]

A laminated photosensitive material No. 3 was prepared in the same manner as in the photosensitive material Example 1 but using the indole derivative (example compound 22) of the formula (19) obtained in Synthesis Example 3 instead of using the charge transporting agent used in the photosensitive material Example 1.

### [Photosensitive Material Comparative Example 1]

A laminated photosensitive material No. 4 was prepared in the same manner as in the photosensitive material Example 1 but using an N-phenyl-substituted indole compound(comparative compound 1) of the following formula instead of using the charge transporting agent used in the photosensitive material Example 1.
Here, the N-phenyl-substituted indole compound (comparative compound No. 1) of the following formula was the same compound as the indole compound of the formula (15) used as a starting material in the Synthesis Example 1.

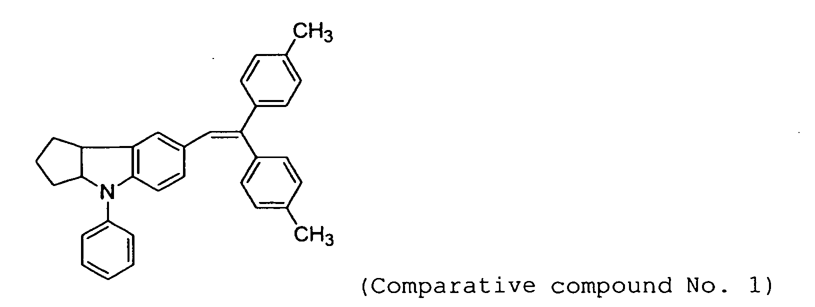

### (Evaluating the electrophotographic properties of the photosensitive materials)

The photosensitive materials prepared in the photosensitive material Examples 1 to 3 and in the photosensitive material Comparative Example 1 were evaluated for their electrophotographic properties by using an electrostatic copying paper testing apparatus (trade name "EPA-8100A").
First, the photosensitive material was subjected to a corona discharge of -5.5 kV in a dark place, and a charged potential V0 at this moment was measured.
Next, the photosensitive material was exposed to monochromatic light of 780 nm of 1.0 µ W/cm² and was found for its half-decay exposure E1/2 (µ J/cm²) and a residual potential Vr (-V) after exposed to light for 2 seconds. The results were as shown in Table 1.

**Table 1**

| Ex. and Comp. Ex. | Photosensitive material No. | Charged potential VO(-V) | Half decay exposure E1/2 (µJ/cm²) | Residual potential Vr(-V) |
|---|---|---|---|---|
| Ex.1 | 1 | 752 | 0.25 | 26 |
| Ex.2 | 2 | 749 | 0.25 | 23 |
| Ex.3 | 3 | 746 | 0.24 | 22 |
| Comp. Ex. 1 | 4 | 775 | 0.26 | 40 |

From the above table, it will be learned that the photosensitive materials for electrophotography containing the indole derivative of the general formula (1) of the present invention as a material of the charge transporting layer, have low residual potentials.

### [Photosensitive Material Example 4]

As a charge generating agent, there was provided a titanylphthalocyanine (charge generating agent No. 2) having intense peaks at the diffraction angles 2 θ ± 0.2° of 7.5, 10.3, 12.6, 22.5, 24.3, 25.4 and 28.6 in the X-ray diffraction spectrum of Cu-Kα.
As a resin binder, further, a polyvinyl butyral resin (S-LEC BL-S manufactured by Sekisui Kagaku Kogyo Co.) was provided.
1.5 Parts by mass of the above charge generating agent (No. 2) was added to 50 parts by mass of a cyclohexanone solution containing 3% of the above polyvinyl butyral resin, and was dispersed therein for one hour by using an ultrasonic wave dispersing machine to prepare a coating solution for forming a charge generating layer.
The obtained coating solution was applied onto the aluminum surface of an aluminum-deposited PET film (electrically conducting substrate) by using the wire bar, and was dried under normal pressure at 110°C for 1 hour to form a charge generating layer of a thickness of 0.2 µ m.

As the charge transporting agent, on the other hand, the indole derivative (example compound 4) of the formula (17) obtained in the Synthesis Example 1 was provided.
As the binder resin for the charge transporting layer, further, a polycarbonate resin (Iupilon Z manufactured by Mitsubishi Engineering Plastic Co.) was provided.

0.9 Parts by mass of the above charge transporting agent was added to 7.38 parts by mass of a tetrahydrofuran solution containing 12.2% of the above polycarbonate resin, and the charge transporting agent (indole derivative of the formula (17)) was completely dissolved therein by applying ultrasonic waves. The solution was applied onto the charge generating layer by using the wire bar and was dried under normal pressure at 110°Cfor 30 minutes to form a charge transporting layer of a thickness of 10 µ m. Further, a semitransparent gold electrode was deposited on the charge transporting layer to prepare a laminated photosensitive material No. 5.

### [Photosensitive Material Example 5]

A laminated photosensitive material No. 6 was prepared in the same manner as in the photosensitive material Example 4 but using the indole derivative (example compound 5) of the formula (18) obtained in Synthesis Example 2 instead of using the charge transporting agent used in the photosensitive material Example 4.

### [Photosensitive Material Example 6]

A laminated photosensitive material No. 7 was prepared in the same manner as in the photosensitive material Example 4 but using the indole derivative (example compound 22) of the formula (19) obtained in Synthesis Example 3 instead of using the charge transporting agent used in the photosensitive material Example 4.

### [Photosensitive Material Comparative Example 2]

A laminated photosensitive material No. 8 was prepared in the same manner as in the photosensitive material Example 4 but using the N-phenyl-substituted indole compound (comparative compound 1) used as the starting material in the Synthesis Example 1 instead of using the charge transporting agent used in the photosensitive material Example 4.

### [Evaluating the drift mobility]

The photosensitive materials prepared in the photosensitive material Examples 4 to 6 and in the photosensitive material Comparative Example 2 were measured for their drift mobilities. The measurement was taken by the time-of-flight method at 2 × 10⁵ V/cm. The results were as shown in Table 2.

**Table 2**

| Ex. and Comp. Ex. | Photosensitive material No. | Drift mobility [cm²/V·s] |
|---|---|---|
| Ex. 4 | 5 | 1.8 × 10⁻⁵ |
| Ex. 5 | 6 | 2.0 × 10⁻⁵ |
| Ex. 6 | 7 | 3.3 × 10⁻⁵ |
| Comp. Ex. 2 | 8 | 6.4 × 10⁻⁶ |

From the above table, it will be learned that the indole derivatives represented by the general formula (1) of the present invention have high carrier mobilities.

### Industrial Applicability:

The indole derivatives of the invention have high carrier mobilities and have excellent properties for use as charge transporting agents. When an organic photosensitive material for electrophotography is prepared by using them as the charge transporting agent, therefore, there can be obtained favorable electrophotographic properties featuring high sensitivity and low residual potential.

## Claims

1. An indole derivative represented by the following general formula (1), 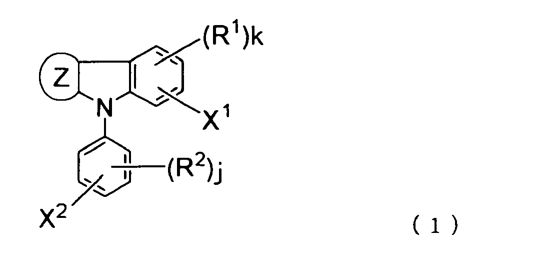 wherein,
R¹ and R² may be same or different, and are groups selected from the group consisting of an alkyl group having 1 to 6 carbon atoms; an alkoxy group having 1 to 6 carbon atoms; a halogen atom; an aromatic hydrocarbon group; an aromatic heterocyclic group; a condensed polycyclic aromatic group; and a di-substituted amino group which has, as a substituent, an alkyl group with 1 to 6 carbon atoms, an alkenyl group with 1 to 6 carbon atoms, an aralkyl group, an aromatic hydrocarbon group or an aromatic heterocyclic group;,
k is an integer of 0 to 3,
j is an integer of 0 to 4,
(when k or j is an integer of not smaller than 2, a plurality of R¹s or R²s may be different from each other),
a ring Z bonded to the indoline ring is a 5- to 8-membered ring having no unsaturated bond in the ring, and may have nitrogen and/or oxygen as ring-constituting atoms,
X¹ is a monovalent group represented by the following general formula (1a),
-(-CR³=CR⁴-)ₘ-CR⁵=CR⁶R⁷ (1a)
wherein,
m is 0 or 1, and
R³ to R⁷ may be same or different, and are hydrogen atoms, alkyl groups having 1 to 6 carbon atoms, alkoxy groups having 1 to 6 carbon atoms, aromatic hydrocarbon groups, aromatic heterocyclic groups or condensed polycyclic aromatic groups, R⁶ and R⁷ together may form a ring, and-when R⁶ is a hydrogen atom or an alkyl group, R⁷ is an aromatic hydrocarbon group, an aromatic heterocyclic group or a condensed polycyclic aromatic group, and
x² is a monovalent group represented by the following general formula (1b),
-(-CR⁸=CR⁹-)ₙ-CR¹⁰=CR¹¹R¹² (1b)
wherein,
n is 0 or 1, and
R⁸ to R¹² may be same or different, and are hydrogen atoms, alkyl groups having 1 to 6 carbon atoms, alkoxy groups having 1 to 6 carbon atoms, aromatic hydrocarbon groups, aromatic heterocyclic groups or condensed polycyclic aromatic groups, R¹¹ and R¹² together may form a ring, and when R¹¹ is a hydrogen atom or an alkyl group, R¹² is an aromatic hydrocarbon group, an aromatic heterocyclic group or a condensed polycyclic aromatic group.

2. The indole derivative according to claim 1, wherein in the general formula (1), the ring Z is a 5-membered ring with carbon atoms as ring-constituting atoms.

3. The indole derivative according to claim 2, wherein in the general formula (1a) representing the group X¹, m is 0 and in the general formula (1b) representing the group X², n is 0.

4. The indole derivative according to claim 2, wherein in the general formula (1a) representing the group X¹, m is 0 and in the general formula (1b) representing the group X², n is 1.

5. The indole derivative according to claim 2, wherein in the general formula (1), k and j are 0s.

6. A charge transporting agent comprising the indole derivative of claim 1.

7. An organic photosensitive material for electrophotography comprising an organic photosensitive layer provided on an electrically conducting substrate, said organic photosensitive layer containing the indole derivative of claim 1 as a charge transporting agent.

8. The organic photosensitive material for electrophotography according to claim 7, wherein said organic photosensitive layer is a lamination type photosensitive layer which comprises a charge generating layer in which the charge generating agent is dispersed in a resin binder and a charge transporting layer in which the charge transporting agent is dispersed in a resin binder.

9. The organic photosensitive material for electrophotography according to claim 7, wherein said organic photosensitive layer is a single layer type photosensitive layer in which the charge generating agent and the charge transporting agent are dispersed in a resin binder.
